(19) Europäisches Patentamt / European Patent Office / Office européen des brevets

(11) EP 4 635 577 A2

(12) EUROPEAN PATENT APPLICATION

(43) Date of publication:
**22.10.2025 Bulletin 2025/43**

(21) Application number: **25200814.9**

(22) Date of filing: **13.10.2020**

(51) International Patent Classification (IPC):
***A61Q 17/04*** *(2006.01)*

(52) Cooperative Patent Classification (CPC):
**A61K 8/31; A61K 8/062; A61K 8/922; A61K 8/925; A61K 8/927; A61Q 17/04;** A61K 2800/412

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.10.2019 US 201962914721 P**

(62) Document number(s) of the earlier application(s) in accordance with Art. 76 EPC:
**20804028.7 / 4 044 992**

(71) Applicant: **Lubrizol Advanced Materials, Inc.**
**Cleveland, OH 44141-3247 (US)**

(72) Inventors:
- **BIRJANDI-NEJAD, Hossein**
**Virginia, 22101 (US)**
- **BLASCO, Laurent**
**83910 Pourrieres (FR)**
- **MORAN, Bryan P.**
**Cleveland, 44141-3247 (US)**
- **LUBNIN, Alexander V.**
**Cleveland, 44141-3247 (US)**

(74) Representative: **Bradley, Josephine Mary et al**
**Kilburn & Strode LLP**
**Lacon London**
**84 Theobalds Road**
**London WC1X 8NL (GB)**

Remarks:
This application was filed on 08-09-2025 as a divisional application to the application mentioned under INID code 62.

# (54) WATER-RESISTANT AND/OR PHOTOPROTECTIVE COMPOSITIONS COMPRISING NON-SOLUBILIZED MICRONIZED WAXES

(57) Provided are water-resistant and/or photoprotective compositions including: (a) an aqueous phase including from 0% to 99.9% by weight of the composition, based on the total weight of the composition; (b) an oil phase including from 0% to 99.9% by weight of the composition, based on the total weight of the composition; (c) an active sun block agent; and (d) a micronized, non-solubilized wax including 0.1% to 10% by weight of the composition, based on the total weight of the composition; wherein the wax has a D50 particle size of 1 to 100 μm, and a melting point of at least 70 °C; and wherein the composition includes at least 50% by weight of at least one of the aqueous phase or the oil phase. Uses of the compositions, and methods utilizing the compositions, are also provided.



Processed by Luminess, 75001 PARIS (FR)

## Description

### TECHNICAL FIELD

The disclosed technology relates to novel cosmetic/dermatologic compositions, such as for topical application, for ultraviolet (UV)-photoprotection of skin, scalp, lips, mucous membranes and/or hair against the damaging effects of UV irradiation, such as solar radiation. For example, the disclosed technology relates to novel water-resistant and sun protection factor (SPF)-improved sunscreen formulations containing non-solubilized, micronized, waxes, such as natural waxes, for example, carnauba wax, rice bran wax, sunflower wax, castor wax or combinations thereof.

### BACKGROUND

UV radiation, consisting of light wavelengths of from 10 to 400 nm, is categorized into three major subtypes (according to the International Organization for Standardization's ISO-21348 standard): UVA (315-400 nm), UVB (280-315 nm) and, UVC (100-280 nm). While UVA and UVB rays are transmitted through the atmosphere, all UVC and some UVB rays are absorbed by the Earth's ozone layer. So, most of the UV rays humans come in contact with are UVA, with a small amount of UVB. UVB radiation causes erythema and burning of the skin which may impair the development of a natural tan; hence, such UVB radiation should be screened from the skin. UVA radiation, which tans the skin, also adversely affects it, in particular in the case of sensitive skin or skin which is continually exposed to such radiation. UVA radiation may cause, in particular, a loss in the elasticity of the skin and the appearance of wrinkles, promoting a premature aging thereof. Such irradiation promotes triggering of the erythematous reaction or enhances this reaction in certain individuals, and may even be the source of phototoxic or photoallergic reactions. Thus, it is desirable to also screen UVA radiation.

A wide variety of cosmetic compositions intended for UVA and/or UVB photoprotection of human skin have been developed. These photoprotective compositions (sometimes referred to as sunscreen compositions) are often oil-in-water emulsions (i.e., a cosmetically acceptable vehicle, carrier or diluent comprising an aqueous continuous dispersing phase and an oily discontinuous dispersed phase). These compositions contain, in various concentrations, one or more standard lipophilic and/or hydrophilic organic sunscreen compounds capable of selectively absorbing harmful or deleterious UV radiation. These compounds, and the amounts thereof, are selected as a function of the desired sun protection factor, the SPF being expressed mathematically by the ratio of the irradiation time required to attain the erythema-forming threshold with the UV screening agent to the time required to attain the erythema-forming threshold in the absence of UV screening agent.

There exists an increasing demand for higher SPF sunscreen products with using as low a concertation of active UV filters as possible, but which also have high water resistance, since sunscreens are typically used during water activities. However, this is difficult, as high SPF sunscreen compositions contain high levels of sunscreen active agents that result in the formulations possibly having negative or objectionable effect(s) on the product, such as insufficient water resistance, greasiness, tackiness and/or stickiness. Furthermore, certain UV filters may absorb into skin or other substrates, and possibly cause damage, so it may be desirable to include components in a sunscreen composition which prevent the UV filter from absorbing into the substrate, without decreasing SPF. Thus, there remains a need for sun screen compositions with high photoprotection performance (e.g., high SPF value), adequate water resistance with minimal sunscreen concentration for better sensory profile, and/or enhanced safety.

The disclosed technology, therefore, provides compositions with improved SPF and/or water-resistance by incorporating micronized, non-soluble waxes into the compositions.

### SUMMARY

The subject matter disclosed herein provides water-resistant and/or photoprotective compositions comprising: an aqueous phase comprising from 0% to 99.9% by weight of the composition, based on the total weight of the composition; an oil phase comprising from 0% to 99.9% by weight of the composition, based on the total weight of the composition; an active sun block agent; and a micronized, non-solubilized wax comprising 0.1% to 10% by weight of the composition, based on the total weight of the composition; wherein the wax has a melting point of at least 70 °C; and wherein the composition comprises at least 50% (such as 55%, 60%, 65%, 70%, 75%, 80%, 85%, or 90%) by weight of at least one of the aqueous phase or the oil phase. By "aqueous phase" it is meant that the phase may include water and/or alcohol, and optionally at least one humectant as the diluent for the phase.

In certain embodiments, the wax has a D50 particle size of 1 to 100 μm. In certain embodiments, the wax has a D50 particle size of 1 to 50 μm. In certain embodiments, the wax has a D50 particle size of 1 to 20 μm. In certain embodiments, the wax has a D50 particle size of 1 to 10 μm. In certain embodiments, the wax has a D50 particle size of 2 to 50 μm. In certain embodiments, the wax has a D50 particle size of 2 to 20 μm. In certain embodiments, the wax has a D50

particle size of 2 to 10 μm. In certain embodiments, the wax has a D50 particle size of 0.5 μm (such as 1 μm, 2 μm, 3 μm, 4 μm, 5 μm, 6 μm, 7 μm, 8 μm, or 9 μm) to 200 μm (such as 175 μm, 150 μm, 125 μm, 100 μm, 90 μm, 80 μm, 70 μm, 60 μm, 50 μm, 45 μm, 40 μm, 35 μm, 30 μm, 25 μm, 20 μm, 19 μm, 18 μm, 17 μm, 16 μm, 15 μm, 14 μm, 13 μm, 12 μm, 11 μm, or 10 μm). In certain embodiments, the wax has a melting point of 70 °C (such as 75 °C or 80 °C) to 100 °C (such as 95 °C, 90 °C, or 85 °C).

In certain embodiments, the wax has a particle size distribution such that 98% of the particles, by mass, fall within the range of 0.5 μm (such as 1 μm, 2 μm, 3 μm, 4 μm, 5 μm, 6 μm, 7 μm, 8 μm, or 9 μm) to 200 μm (such as 175 μm, 150 μm, 125 μm, 100 μm, 90 μm, 80 μm, 70 μm, 60 μm, 50 μm, 45 μm, 40 μm, 35 μm, 30 μm, 25 μm, 20 μm, 19 μm, 18 μm, 17 μm, 16 μm, 15 μm, 14 μm, 13 μm, 12 μm, 11 μm, or 10 μm). In these embodiments, the particle size distribution may be defined by the D1 and D99 particle size distribution measurements of the wax.

In certain embodiments, the wax has a particle size distribution such that 96% of the particles, by mass, fall within the range of 0.5 μm (such as 1 μm, 2 μm, 3 μm, 4 μm, 5 μm, 6 μm, 7 μm, 8 μm, or 9 μm) to 200 μm (such as 175 μm, 150 μm, 125 μm, 100 μm, 90 μm, 80 μm, 70 μm, 60 μm, 50 μm, 45 μm, 40 μm, 35 μm, 30 μm, 25 μm, 20 μm, 19 μm, 18 μm, 17 μm, 16 μm, 15 μm, 14 μm, 13 μm, 12 μm, 11 μm, or 10 μm). In these embodiments, the particle size distribution may be defined by the D3 and D97 particle size distribution measurements of the wax.

In certain embodiments, the wax has a particle size distribution such that 90% of the particles, by mass, fall within the range of 0.5 μm (such as 1 μm, 2 μm, 3 μm, 4 μm, 5 μm, 6 μm, 7 μm, 8 μm, or 9 μm) to 200 μm (such as 175 μm, 150 μm, 125 μm, 100 μm, 90 μm, 80 μm, 70 μm, 60 μm, 50 μm, 45 μm, 40 μm, 35 μm, 30 μm, 25 μm, 20 μm, 19 μm, 18 μm, 17 μm, 16 μm, 15 μm, 14 μm, 13 μm, 12 μm, 11 μm, or 10 μm). In these embodiments, the particle size distribution may be defined by the D5 and D95 particle size distribution measurements of the wax.

In certain embodiments, the wax has a particle size distribution such that 80% of the particles, by mass, fall within the range of 0.5 μm (such as 1 μm, 2 μm, 3 μm, 4 μm, 5 μm, 6 μm, 7 μm, 8 μm, or 9 μm) to 200 μm (such as 175 μm, 150 μm, 125 μm, 100 μm, 90 μm, 80 μm, 70 μm, 60 μm, 50 μm, 45 μm, 40 μm, 35 μm, 30 μm, 25 μm, 20 μm, 19 μm, 18 μm, 17 μm, 16 μm, 15 μm, 14 μm, 13 μm, 12 μm, 11 μm, or 10 μm). In these embodiments, the particle size distribution may be defined by the D10 and D90 particle size distribution measurements of the wax.

In certain embodiments, the wax comprises 1% to 5% of the composition, based on the total weight of the composition. In certain embodiments, the wax may be present in amounts of from 0.1% (such as 0.2%, 0.3%, 0.4%, 0.5%, 0.6%, 0.7%, 0.8%, 0.9%, or 1%) to 10% (such as 9%, 8%, 7%, 6%, 5%, 4%, 3%, or 2%) by weight, based on the total weight of the composition.

In certain embodiments, the wax comprises a natural wax. In certain embodiments, the wax comprises at least one of beeswax, scale insect waxes, wool wax, spermaceti, liquid marine oils, palm tree wax, candelilla wax, retamo wax, flax wax, cotton wax, hemp wax, sugarcane wax, esparto wax, sorghum-grain wax, rice bran wax, leaf blade wax, waxes from roots, waxes from barks, myrica (fruit) wax, cranberry wax, cuticle waxes of fruit, liquid vegetable waxes, floral waxes, candelilla wax, bayberry wax, japan (sumac) wax, ouricury wax, soy wax, chinese tallow tree wax, carnauba wax, sunflower wax, castor wax, berry wax, or jojoba wax. In certain embodiments, the wax comprises carnauba wax. In certain embodiments, the wax comprises castor wax. In certain embodiments, the wax comprises rice bran wax. In certain embodiments, the wax comprises sunflower wax. In certain embodiments, the compositions comprise an emulsion.

In certain embodiments, the compositions further comprise a stabilizing and/or emulsifying component.

In certain embodiments, the stabilizing and/or emulsifying component is present in the compositions in an amount of 0.1% (such as 0.2%, 0.3%, 0.4%, 0.5%, 0.6%, 0.7%, 0.8%, 0.9%, 1%, 1.5%, 2%, 3%, or 4%) to 10% (such as 9%, 8%, 7%, 6% or 5%) by weight, based on the total weight of the composition.

In certain embodiments, the compositions comprise a water-in-oil emulsion or an oil-in-water emulsion.

In certain embodiments, the active sun block agent comprises at least one of dibenzoylmethane, anthranilate, benzophenone, p-aminobenzoic acid, camphor, cinnamate, salicylate, β,β-diphenylacrylate, triazine, benzimidazole, bis-benzoazolyl, methylene bis-(hydroxyphenylbenzotriazole), polymer sun block agent, or silicone sun block agent.

In certain embodiments, the active sun block agent comprises at least one of avobenzone, octocrylene, or oxybenzone octyl salicylate.

In certain embodiments, the active sun block agent comprises a metal oxide sunscreen. In certain embodiments, the active sun block agent comprises at least one of titanium oxide, silicon dioxide, aluminum oxide, zinc oxide, iron oxide, kaolin, talc, phosphate nanomaterials, carbonate nanomaterials, or hydroxyapatite.

In certain embodiments, the compositions may further comprise an artificial tanning agent. In certain embodiments, the artificial tanning agent may comprise dihydroxyacetone. Artificial tanning agents are materials which provide a tanned look without exposure to harmful UV rays.

In certain embodiments, the compositions described above may be formulated as creams, dispersions, emulsions, gels, ointments, lotions, foams, sprays, or tonics.

Also provided are topically-applicable cosmetic/dermatologic sunscreen oil-in-water emulsion compositions for ultraviolet-photoprotection of skin, scalp, lips, mucous membranes, and/or hair against the damaging effects of ultraviolet irradiation comprising (a) an active sun block agent and (b) a micronized, non-solubilized natural wax, formulated into (c) a

topically-applicable, cosmetically/dermatologically-acceptable vehicle, diluent or carrier.

Also provided are topically-applicable cosmetic/dermatologic formulation compositions for ultraviolet-photoprotection of skin, scalp, lips, mucous membranes, and/or hair against the damaging effects of ultraviolet irradiation comprising (a) an active sun block agent and (b) a micronized, non-solubilized natural wax, formulated into (c) a topically-applicable, cosmetically/dermatologically-acceptable vehicle, diluent or carrier.

Also provided are water-resistant and/or photoprotective compositions comprising: an aqueous phase comprising from 20% to 80% by weight of the composition, based on the total weight of the composition; an oil phase comprising from 5% to 50% by weight of the composition, based on the total weight of the composition, wherein the oil phase comprises an organic active sun block agent; and a micronized, non-solubilized, natural wax comprising 1% to 5% by weight of the composition, based on the total weight of the composition; and a stabilizing and/or emulsifying component comprising 1% to 10% by weight of the composition, based on the total weight of the composition; wherein the wax has a D50 particle size of 2 to 50 μm, and a melting point of 70 to 100 °C.

Also provided are methods of mitigating the effects of ultraviolet irradiation on human skin, scalp, lips, mucous membranes and/or hair by applying the composition(s) described above thereto, in an amount effective to at least partially mitigate the effects of ultraviolet irradiation.

Also provided are uses of the compositions described above for ultraviolet-photoprotection of human skin, scalp, lips, mucous membranes and/or hair against the damaging effects of ultraviolet irradiation.

Also provided are uses of micronized, non-solubilized waxes in sunscreen compositions, for improving the aesthetics and/or sun protection factor of the sunscreen compositions.

The following embodiments of the present subject matter are contemplated:

1. A water-resistant and/or photoprotective composition comprising: an aqueous phase comprising from 0% to 99.9% by weight of the composition, based on the total weight of the composition; an oil phase comprising from 0% to 99.9% by weight of the composition, based on the total weight of the composition; an active sun block agent; and a micronized, non-solubilized wax comprising 0.1% to 10% by weight of the composition, based on the total weight of the composition; wherein the wax has a D50 particle size of 1 to 100 μm, and a melting point of at least 70 °C; and wherein the composition comprises at least 50% by weight of at least one of the aqueous phase or the oil phase.
2. The composition of embodiment 1, wherein the wax has a D50 particle size of 1 to 50 μm.
3. The composition of either embodiment 1 or embodiment 2, wherein the wax has a D50 particle size of 1 to 20 μm.
4. The composition of any one of embodiments 1 to 3, wherein the wax has a D50 particle size of 1 to 10 μm.
5. The composition of either embodiment 1 or embodiment 2, wherein the wax has a D50 particle size of 2 to 50 μm.
6. The composition of any one of embodiments 1 to 3, wherein the wax has a D50 particle size of 2 to 20 μm.
7. The composition of any one of embodiments 1 to 4, wherein the wax has a D50 particle size of 2 to 10 μm.
8. The composition of any one of embodiments 1 to 7, wherein the wax has a melting point of 70 to 100 °C.
9. The composition of any one of embodiments 1 to 8, wherein the wax comprises 1% to 5% of the composition, based on the total weight of the composition.
10. The composition of any one of embodiments 1 to 9, wherein the wax comprises a natural wax.
11. The composition of any one of embodiments 1 to 10, wherein the wax comprises at least one of beeswax, scale insect waxes, wool wax, spermaceti, liquid marine oils, palm tree wax, candelilla wax, retamo wax, flax wax, cotton wax, hemp wax, sugarcane wax, esparto wax, sorghum-grain wax, rice bran wax, leaf blade wax, waxes from roots, waxes from barks, myrica (fruit) wax, cranberry wax, cuticle waxes of fruit, liquid vegetable waxes, floral waxes, candelilla wax, bayberry wax, japan (sumac) wax, ouricury wax, soy wax, chinese tallow tree wax, carnauba wax, sunflower wax, castor wax, berry wax, or jojoba wax.
12. The composition of any one of embodiments 1 to 11, wherein the wax comprises carnauba wax.
13. The composition of any one of embodiments 1 to 11, wherein the wax comprises castor wax.
14. The composition of any one of embodiments 1 to 11, wherein the wax comprises rice bran wax.
15. The composition of any one of embodiments 1 to 11, wherein the wax comprises sunflower wax.
16. The composition of any one of embodiments 1 to 15, wherein the composition comprises an emulsion.
17. The composition of any one of embodiments 1 to 16, wherein the composition further comprises a stabilizing and/or emulsifying component.
18. The composition of embodiment 17, wherein the stabilizing and/or emulsifying component is present in the composition in an amount of 0.1% to 10% by weight, based on the total weight of the composition.
19. The composition of either embodiment 17 or embodiment 18, wherein the composition comprises a water-in-oil emulsion or an oil-in-water emulsion.
20. The composition of any one of embodiments 1 to 19, wherein the active sun block agent comprises at least one of dibenzoylmethane, anthranilate, benzophenone, p-aminobenzoic acid, camphor, cinnamate, salicylate, β,β-diphenylacrylate, triazine, benzimidazole,
bis-benzoazolyl, methylene bis-(hydroxyphenylbenzotriazole), polymer sun block agent, or silicone sun block agent.

21. The composition of any one of embodiments 1 to 19, wherein the active sun block agent comprises at least one of avobenzone, octocrylene, or oxybenzone octyl salicylate.
22. The composition of any one of embodiments 1 to 21, wherein the active sun block agent comprises a metal oxide sunscreen.
23. The composition of any one of embodiments 1 to 22, wherein the active sun block agent comprises at least one of titanium oxide, silicon dioxide, aluminum oxide, zinc oxide, iron oxide, kaolin, talc, phosphate nanomaterials, carbonate nanomaterials, or hydroxyapatite.
24. The composition of any one of embodiments 1 to 23, wherein the composition further comprises an artificial tanning agent.
25. The composition of embodiment 24, wherein the artificial tanning agent comprises dihydroxyacetone.
26. The composition of any one of embodiments 1 to 25, formulated as a cream, dispersion, emulsion, gel, ointment, lotion, foam, spray, or tonic.
27. A topically-applicable cosmetic/dermatologic sunscreen oil-in-water emulsion composition for ultraviolet-photoprotection of skin, scalp, lips, mucous membranes, and/or hair against the damaging effects of ultraviolet irradiation comprising (a) an active sun block agent and (b) a micronized, non-solubilized natural wax, formulated into (c) a topically-applicable, cosmetically/dermatologically-acceptable vehicle, diluent or carrier.
28. A topically-applicable cosmetic/dermatologic formulation composition for ultraviolet-photoprotection of skin, scalp, lips, mucous membranes, and/or hair against the damaging effects of ultraviolet irradiation comprising (a) an active sun block agent and (b) a micronized, non-solubilized natural wax, formulated into (c) a topically-applicable, cosmetically/dermatologically-acceptable vehicle, diluent or carrier.
29. A water-resistant and/or photoprotective composition comprising: an aqueous phase comprising from 20% to 80% by weight of the composition, based on the total weight of the composition; an oil phase comprising from 5% to 50% by weight of the composition, based on the total weight of the composition, wherein the oil phase comprises an organic active sun block agent; and a micronized, non-solubilized, natural wax comprising 1% to 5% by weight of the composition, based on the total weight of the composition; and a stabilizing and/or emulsifying component comprising 1% to 10% by weight of the composition, based on the total weight of the composition; wherein the wax has a D50 particle size of 2 to 50 μm, and a melting point of 70 to 100 °C.
30. A method of mitigating the effects of ultraviolet irradiation on human skin, scalp, lips, mucous membranes and/or hair by applying the composition of any one of embodiments 1 to 29 thereto, in an amount effective to at least partially mitigate the effects of ultraviolet irradiation.
31. Use of the composition of any one of embodiments 1 to 29 for ultraviolet-photoprotection of human skin, scalp, lips, mucous membranes and/or hair against the damaging effects of ultraviolet irradiation.
32. Use of a micronized, non-solubilized wax in a sunscreen composition for improving the aesthetics and/or sun protection factor of the sunscreen composition.

## BRIEF DESCRIPTION OF THE FIGURES

FIG. 1 illustrates the modes of action of organic and inorganic UV filters.
FIG. 2 depicts an illustrative wax particle according to the present disclosure, having irregular shape and sharp edges.
FIG. 3 depicts an illustrative particle size distribution chart.

## DETAILED DESCRIPTION

Various features and embodiments of the present subject matter will be described below by way of non-limiting illustration.

The amount of each chemical component described herein is presented exclusive of any solvent or diluent oil, which may be customarily present in the commercial material, that is, on an active chemical basis, unless otherwise indicated. However, unless otherwise indicated, each chemical or composition referred to herein should be interpreted as being a commercial grade material which may contain the isomers, by-products, derivatives, and other such materials which are normally understood to be present in the commercial grade.

It is known that some of the materials described herein may interact in the final formulation, so that the components of the final formulation may be different from those that are initially added. For instance, metal ions (of, e.g., a detergent) may migrate to other acidic or anionic sites of other molecules. The products formed thereby, including the products formed upon employing the composition of the present subject matter in its intended use, may not be susceptible of easy description. Nevertheless, all such modifications and reaction products are included within the scope of the present subject matter; the present subject matter encompasses the composition prepared by admixing the

components described herein.

As used herein, the indefinite article "a" is intended to mean one or more than one. As used herein, the phrase "at least one" means one or more than one of the following term(s). Thus, "a" and "at least one" may be used interchangeably. For example, "at least one of A, B or C" means that just one of A, B or C may be included, and any mixture of two or more of A, B and C may be included, in alternative embodiments. As another example, "at least one X" means that one or more than one material/component X may be included.

As used herein, the term "about" means that a value of a given quantity is within ±20% of the stated value. In other embodiments, the value is within ±15% of the stated value. In other embodiments, the value is within ±10% of the stated value. In other embodiments, the value is within ±5% of the stated value. In other embodiments, the value is within ±2.5% of the stated value. In other embodiments, the value is within ±1% of the stated value. In other embodiments, the value is within a range of the explicitly-described value which would be understood by those of ordinary skill, based on the disclosures provided herein, to perform substantially similarly to compositions including the literal amounts described herein.

As used herein, the term "substantially" means that a value of a given quantity is within ±10% of the stated value. In other embodiments, the value is within ±5% of the stated value. In other embodiments, the value is within ±2.5% of the stated value. In other embodiments, the value is within ±1% of the stated value.

As used herein, the term "substantially free of" means that a component does not include any intentional addition of the material which the component is "substantially free of". For example, the component may include a material which the component is "substantially free of" at no more than impurity levels, which may be the result of incomplete chemical reactions and/or unintended/undesired (but perhaps unavoidable) reaction products.

As used herein, the transitional term "comprising," which is synonymous with "including," "containing," or "characterized by," is inclusive or open-ended and does not exclude additional, un-recited elements or method steps. However, in each recitation of "comprising" herein, it is intended that the term also encompass, as alternative embodiments, the phrases "consisting essentially of" and "consisting of," where "consisting of" excludes any element or step not specified and "consisting essentially of" permits the inclusion of additional un-recited elements or steps that do not materially affect the essential or basic and novel characteristics of the composition or method under consideration.

As used herein, the phrase "active sun block agent" means any material which provides protection from UV rays at a sun protection factor of 2 or greater. The phrases "UV filter", "UV blocker", "UV absorber", "UV-photoprotecting agent", "sunscreen agent", and like terms, are used synonymously with the phrase "active sun block agent", and should not be construed as limiting to any particular type of sun block agent, unless explicitly stated or required by context.

It has now surprisingly and unexpectedly been determined that by formulating non-solubilized micronized waxes into sunscreen compositions, the final product formulations feature high SPF value and excellent water resistance when topically applied onto skin, scalp, lips, mucous membranes and/or hair, such as when compared to identical formulations not containing micronized waxes. It has also unexpectedly and surprisingly been determined that the addition of micronized, non-solubilized waxes increased the in vitro SPF and water resistance of sunscreen compositions.

More particularly, according to the present subject matter, water-resistant and photoprotective sunscreen formulations exhibiting enhanced SPF may be provided by incorporating therein a non-solubilized, micronized, natural wax, such as carnauba wax and/or rice bran wax. These phenomena were unexpected, as previously the SPF of a sunscreen product was improved, in certain compositions, via use of solubilized waxes. However, we unexpectedly found out that non-solubilized (natural) micronized waxes not only increase SPF value of sunscreen compositions, but also improve water resistance of the formulations.

The micronized waxes according to the present subject matter may be non-solubilized, micronized, natural waxes with irregular particle shape, prepared using air-jet milling or hot-air spray methods. The D50 particle size of the subject waxes may range up to 100 μm, but may desirably be on the order of 2 to 50 μm measured using laser diffraction method.

The shape of the wax particles may be spherical, ellipsoidal, oval, irregular, with no particular shape, and/or with sharp edges. The terms "spherical" or ellipsoidal" or "oval" as used herein also mean that the wax particle has a uniform and substantially spherical or ellipsoidal or oval shape. The term "irregular" as used herein means that the wax particles have no particular shape and they cannot be characterized as spherical, ellipsoidal or oval. The term "sharp edges" mean that the wax particles have geometry with internal angle of less than 90 °, or less than 45 $^0$ as shown in FIG. 2. The term "substantially" as used in the context of the shape of a spherical particle means that the particle is of substantially isotropic shape, i.e., it has a relatively regular morphology. The term "particle size" as used herein refers to the volume equivalent diameter of a single particle. In other words, dimeter of an irregular particle is reported as the dimeter of a spherical shape with a volume equivalent to the volume of the irregular particle.

The wax may be used in the sunscreen compositions in amounts of from 0.1% to 10% by weight, such as 1% to 5% by weight, based on the total weight of the composition. In certain embodiments, the wax may be present in amounts of from 0.1% (such as 0.2%, 0.3%, 0.4%, 0.5%, 0.6%, 0.7%, 0.8%, 0.9%, or 1%) to 10% (such as 9%, 8%, 7%, 6%, 5%, 4%, 3%, or 2%) by weight, based on the total weight of the composition.

By "micronized", it is meant that the wax undergoes a process which creates wax particles of a size in the micron (μm) range (roughly 1 to 1000 μm). The particle size may be tailored to a certain extent by altering processing parameters. Methods of manufacturing micronized waxes are known in the art, and include the following.

Fluidized bed jet mills and melt spraying are the two most commonly used methods to produce micronized powder. Particle-to-particle collisions occur when polymers pass through opposing jets of high-pressure air in fluidized bed jet mills, producing fine powdered wax polymers. Controlling the flow rate through the milling chamber and the pressure of the opposing jets enables micronization of a wide range of waxy polymers. In-line classifiers are used to adjust and control the resultant particle size distribution into the targeted range. Melt spraying techniques generate fine, spherical wax particles when molten waxy polymers are sprayed into a cooling chamber. Particles are separated from the gas stream in a cyclone chamber or filter bag house. Polymers and polymer blends with broad melting range typically process more efficiently, yielding a narrower particle size distribution when micronized with a fluidized bed jet mill compared to melt spray methods.

Air Jet Mill: Many of the micronized waxes described herein were prepared by an air jet milling process. This process uses high speed jets of compressed air or inert gas to impact particles into each other. The jet mill used to prepare these wax products is designed to output particles below a certain size, while continuing to mill particles above that size, resulting in a narrow size distribution of the resulting product. Particles leaving the mill are separated from the gas stream by a rotating classifier wheel integrated in the grinding chamber of the jet mill. Final particle size is essentially controlled by the rotational speed of the classifier wheel.

Hot Melt Spray: Hot melt extrusion is a solvent-free process that utilizes heat and pressure to disperse the wax molecularly. Typically, solid particles are first heated above their melting point and then sprayed in cool air with applied pressure to solidify.

By "non-solubilized", it is meant that the waxes are not dissolved and/or melted during processing of the compositions/formulations described herein. In other words, the waxes may be dissolved and/or melted to manufacture them into their micronized form, but thereafter are not again dissolved and/or melted before or during manufacture of the compositions described herein.

By "natural", it is meant that the waxes are natural or are derived from natural sources, as opposed to synthetic waxes, which may be manufactured via chemical reactions to form (polymer) waxes.

Suitable waxes include, but are not limited to, at least one of beeswax, scale insect waxes, wool wax, spermaceti, liquid marine oils, palm tree wax, candelilla wax, retamo wax, flax wax, cotton wax, hemp wax, sugarcane wax, esparto wax, sorghum-grain wax, rice bran wax, leaf blade wax, waxes from roots, waxes from barks, myrica (fruit) wax, cranberry wax, cuticle waxes of fruit, liquid vegetable waxes, floral waxes, candelilla wax, bayberry wax, japan (sumac) wax, ouricury wax, soy wax, chinese tallow tree wax, carnauba wax, sunflower wax, castor wax, berry wax, or jojoba wax. In certain embodiments, the wax may comprise at least one of carnauba wax, rice bran wax, sunflower wax, or castor wax.

The non-solubilized, micronized (natural) waxes of the present disclosure may be heterogenous irregular microparticles with sharp edges, and the particles may be non-spheroidal in shape. The particles may be homogeneously distributed throughout a sunscreen formulation. They may be added to oil phase, water phase or post-added to a formulation.

In certain embodiments, cosmetic/dermatologic compositions according to the present disclosure may contain (a) an active sun block agent (such as at least one UVA and/or UVB sunscreen) and (b) a non-solubilized, micronized, natural wax, formulated into (c) a topically applicable, cosmetically/dermatologically acceptable vehicle, diluent or carrier therefor.

By "UVA and/or UVB sunscreen", it is meant any compound or any combination of compounds which, by mechanisms that are known per se of absorption and/or reflection and/or scattering of UVA and/or UVB radiation, prevents, or at least limits, the contact between such radiation and a surface (skin, hair) on which this or these compounds have been applied. Stated differently, these compounds may be UV-absorbing organic screening agents or inorganic (nano)pigments which scatter, absorb and/or reflect UV radiation, as well as mixtures thereof. In certain embodiments, the at least one UVA and/or UVB sunscreen may comprise one or more hydrophilic organic screening agents and/or one or more lipophilic organic screening agents and/or one or more mineral or inorganic (nano)pigments.

A suitable UV-photoprotecting agent is octocrylene, which has the chemical formula:

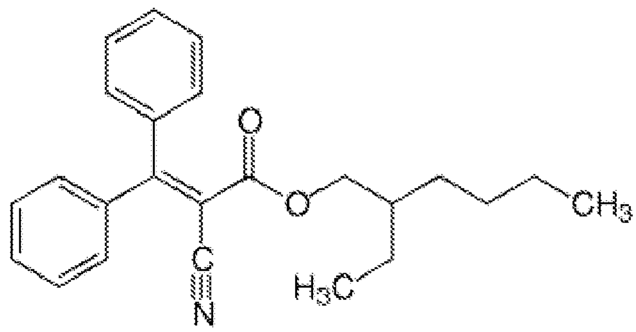

Another suitable UV-photoprotecting agent is homosalate, which has the chemical formula:

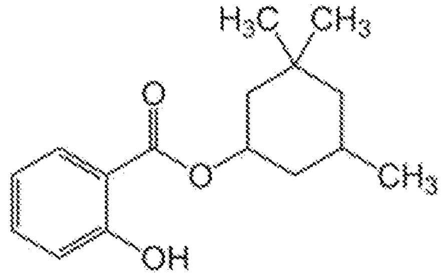

Another suitable UV-photoprotecting agent is ethylhexyl salicylate, which has the chemical formula:

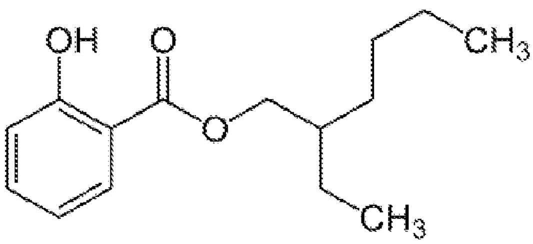

Another suitable UV-photoprotecting agent is the dibenzoylmethane sunscreen avobenzone, or 4-(tert-butyl)-4-methoxydibenzoylmethane, which has the chemical formula:

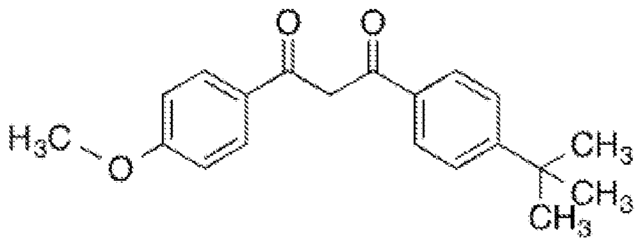

Other suitable UV-photoprotecting agents are metal oxide sunscreens, such as zinc oxide and/or titanium oxide. Sunscreens according to the present disclosure which are physical blockers reflect or scatter ultraviolet radiation. Examples of physical blockers include red petrolatum, titanium dioxide, zinc oxide, iron oxide, kaolin, ichthammol, red veterinary petrolatum, talc, calaminein, phosphate- and carbonate-based nanomaterials, and pure and/or doped hydroxyapatite.

Sunscreens which are chemical absorbers, such as avobenzone, absorb harmful ultraviolet radiation. Chemical absorbers are classified, depending on the type of radiation they protect against, as either UVA or UVB absorbers. UVA absorbers generally absorb radiation in the 320 to 400 nm region of the ultraviolet spectrum. UVA absorbers include anthranilates, benzophenones, and dibenzoyl methanes. UVB absorbers generally absorb radiation in the 280 to 320 nm region of the ultraviolet spectrum. UVB absorbers include p-aminobenzoic acid derivatives, camphor derivatives, cinnamates, and salicylates.

Suitable sunscreens may comprise chemical absorbers, but may also comprise physical blockers. The

mechanisms of organic UV filters vs. inorganic UV filters is provided in Manaia, Eloisa Berbel, et al. "Inorganic UV filters." Brazilian journal of pharmaceutical sciences 49.2 (2013): 201-209. There are two groups of active molecules that may act as UV filters: organic and inorganic. The inorganic UV filters, such as zinc oxide, titanium dioxide, iron oxide, kaolin, ichthammol, red veterinary petrolatum, talc, calaminein, phosphate- and carbonate-based nanomaterials, and pure and/or doped hydroxyapatite, scatter, reflect and/or absorb the sun's potentially harmful UV-radiation that reaches the skin. The organic UV filters may be aromatic compounds with a carbonyl group. On receiving the energy of UV photons, the organic UV filters may act in three ways: (i) undergo conformational molecular changes, (ii) emit radiation at higher wavelength, or (iii) release incident energy as heat. The action mode of the organic protector molecules is reversible, so that the same molecule may function repeatedly. Examples of organic sunscreens are salicylates, cinnamates, benzophenones, anthranilates, dibenzoylmethanes and p-aminobenzoates. FIG. 1 illustrates the modes of action of organic and inorganic UV filters.

Exemplary sunscreens which may be formulated into the compositions of the present disclosure are chemical absorbers such as p-aminobenzoic acid derivatives, anthranilates, benzophenones, camphor derivatives, cinnamic derivatives, dibenzoyl methanes, β,β-diphenylacrylate derivatives, salicylic derivatives, triazine derivatives, benzimidazole compounds, bis-benzoazolyl derivatives, methylene bis-(hydroxyphenylbenzotriazole) compounds, sunscreen polymers and/or silicones, or mixtures thereof.

Exemplary sunscreens which may be formulated into the compositions of the present disclosure are physical blockers such as cerium oxides, chromium oxides, cobalt oxides, iron oxides, red petrolatum, silicone-treated titanium dioxide, titanium dioxide, zinc oxide, zirconium oxide, or mixtures thereof.

Suitable sunscreens active in the UVA and/or UVB range include one or combinations of the following:

- p-aminobenzoic acid,
- oxyethylene (25 mol) p-aminobenzoate,
- 2-ethylhexyl p-dimethylaminobenzoate,
- ethyl N-oxypropylene p-aminobenzoate,
- glycerol p-aminobenzoate,
- 4-isopropylbenzyl salicylate,
- Butyloctyl salicylate and other salicylate-derived UV absorbers,
- Methoxycrylene,
- 2-ethylhexyl 4-methoxycinnamate,
- methyl diisopropylcinnamate,
- isoamyl 4-methoxycinnamate,
- diethanolamine 4-methoxycinnamate,
- 3-(4'-trimethylammunium)-benzyliden-bornan-2-one methylsulfate,
- 2-hydroxy-4-methoxybenzophenone,
- 2-hydroxy-4-methoxybenzophenone-5-sulfonate,
- 2,4-dihydroxybenzophenone,
- 2,2',4,4'-tetrahydroxybenzophenone,
- 2,2'-dihydroxy-4,4'dimethoxybenzophenone,
- 2-hydroxy-4-n-octoxybenzophenone,
- 2-hydroxy-4-methoxy-4'-methoxybenzophenone,
- a-(2-oxoborn-3-ylidene)-tolyl-4-sulfonic acid and soluble salts thereof,
- 3-(4'-sulfo)benzyliden-bornan-2-one and soluble salts thereof,
- 3-(4'methylbenzylidene)-d,1-camphor,
- 3-benzylidene-d,1-camphor,
- benzene 1,4-di(3-methylidene-10-camphosulfonic) acid and salts thereof (the product Mexoryl SX described in U.S. Patent No. 4,585,597 issued to Lange et al. on April 29, 1986),
- urocanic acid,
- 2,4,6-tris[p-(2'-ethylhexyl-1'-oxycarbonyl)-anilino]-1,3,5-triazine,
- 2-[(p-(tertiobutylamido)anilino]-4,6-bis-[(p-(2'-ethylhexyl-1'-oxycarbonyl)anilino]-1,3,5-triazine,
- 2,4-bis {[4-(2-ethyl-hexyloxy)]-2-hydroxy]-phenyl}-6-(4-methoxy-phenyl)-1,3,5-triazine ("TINOSORB S" marketed by Ciba),
- Tris-Biphenyl Triazine (and) Aqua (and) Decyl Glucoside (and) Butylene Glycol (and) Disodium Phosphate (and) Xanthan Gum ("TINOSORB® A2B" marketed by BASF),
- the polymer of N-(2 et 4)-[(2-oxoborn-3-yliden)methyl]benzyl]-acrylamide,
- 1,4-bisbenzimidazolyl-phenylen-3,3',5,5'-tetrasulfonic acid and salts thereof,
- the benzalmalonate-substituted polyorganosiloxanes,
- the benzotriazole-substituted polyorganosiloxanes (Drometrizole Trisiloxane),

- dispersed 2,2'-methylene-bis-[6-(2H-benzotriazol-2-yl)-4-(1,1,3,3-tetramethylbutyl)phenol] such as that marketed under the trademark MIXXIM BB/100 by Fairmount Chemical, or micronized in dispersed form thereof such as that marketed under the trademark TINOSORB M by Ciba-Geigy,
- Solubilized 2,2'-methylene-bis-[6-(2H-benzotriazol-2-yl)-4-(methyl)phenol] such as that marketed under the trademark MIXXIM BB/200 by Fairmount Chemical,
- Natural UV absorbers such as Karanja oil or similar nomenclature Pongamia oil, derived from the seeds of the millettia pinnata tree, millettia pinnata, also known as Pongamia pinnata or Pongamia glabra Extract or oil,
- Naturally-inspired UV absorbers, such as sinapate esters and their derivatives, and
- Synthetic non-regulatory approved UV absorbers such as butyloctyl salicylate and other salicylate derived UV absorbers, methoxycrylene.

Suitable sunscreens are one or more of the following: octyl salicylate, octocrylene, and oxybenzone. Dibenzoyl methane derivatives other than avobenzone are also suitable sunscreens according to the present disclosure, and include one or combinations of the following:

- 2-methyldibenzoylmethane,
- 4-methyldibenzoylmethane,
- 4-isopropyldibenzoylmethane,
- 4-tert.-butyldibenzoylmethane,
- 2,4-dimethyldibenzoylmethane,
- 2,5-dimethyldibenzoylmethane,
- 4,4'-diisopropyldibenzoylmethane,
- 4,4'-dimethoxydibenzoylmethane,
- 2-methyl-5-isopropyl-4'-methoxydibenzoylmethane,
- 2-methyl-5-tert.-butyl-4'-methoxydibenzoylmethane,
- 2,4-dimethyl-4'-methoxydibenzoylmethane, and
- 2,6-dimethyl-4-tert.-butyl-4'-methoxydibenzoylmethane.

The subject at least one UVA and/or UVB sunscreen may be formulated into the compositions disclosed herein in amounts ranging from about 0.01% to about 10%, such as from about 0.1 % to about 6%, by weight thereof, based on the total weight of the composition. Of course, depending upon the nature of the particular formulation, higher or lower amounts may be suitable.

In certain embodiments, the composition may further comprise artificial or sunless tanning compositions such as dihydroxyacetone (DHA). A wide variety of artificial tanning agents has been developed. Artificial tanners provide the highly sought-after tanning or darkening response once only available through harmful exposure to ultraviolet radiation. DHA, in particular, has been widely utilized in cosmetics to accomplish artificial tanning of the skin. Proteins of the epidermis have a very high concentration of arginine, lysine, and histidine, and the reaction of skin with DHA to produce an artificial tan takes advantage of this fact. The tanning reaction proceeds through combination with free amino groups in skin proteins, and particularly by combination of DHA with the free guanido group in arginine. Suitable artificial tanning compositions include, but are not limited to, at least one of allose, alpha hydroxy substituted ketones (such as dihydroxyacetone), altrose, arabinose, erythrose, fructose, galactose, glucose, glyceraldehyde, indoles, lactose, mannose, reose, ribose, pentose, sucrose, tallose, or xylose.

The compositions disclosed herein may be formulated into a wide variety of product types, including creams, dispersions, emulsions (oil-in-water, water-in-oil, water-in-oil-in-water, and oil-in-water-in-silicone), gels, ointments, lotions, foams, sprays, tonics, and the like.

The topical cosmetic compositions of the present disclosure may comprise a carrier (vehicle or diluent) or mixture of carriers. The carrier should be cosmetically and/or pharmaceutically acceptable, which reflects that the carrier is suitable for topical application onto the skin, has good aesthetic properties, is compatible with any other components, and will not cause any untoward safety or toxicity concerns. The carriers and additional components used to formulate such products vary with the product type and may be routinely chosen by one skilled in the art.

The compositions disclosed herein may comprise a carrier, or a mixture of carriers, suitable for topical application onto human skin. The carriers may constitute from about 0.5% to about 99.5% by weight, such as from about 5.0% to about 99.5% by weight, or from about 10.0% to about 98.0% by weight, of the composition, based on the total weight of the composition. As used herein, the phrase "suitable for topical application onto human skin" reflects that the carrier does not damage or negatively affect the water-resistance of the composition, or cause irritation to human skin.

Carriers suitable for use with the present compositions include, for example, those used in the formulation of a wide variety of product types, including creams, dispersions, emulsions, gels, lotions, foams, sprays, and tonics.

The carriers used herein may include a wide range of components conventionally used in cosmetic/dermatological compositions. The carriers may contain a solvent to dissolve or disperse the polymer. The carriers may also contain a wide variety of additional materials including, but not limited to, esters (such as isopropyl myristate), halogenated hydrocarbons (such as freons), hydrocarbons (such as decene, hexane, and isobutane), linalool, and volatile silicon derivatives (such as siloxanes, such as phenyl pentamethyl disiloxane, methoxypropyl heptamethyl cyclotetrasiloxane, chloropropyl pentamethyl disiloxane, hydroxypropyl pentamethyl disiloxane, octamethyl cyclotetrasiloxane, decamethyl cyclopentasiloxane, cyclomethicone, dimethicone), and mixtures thereof.

Foams and aerosol sprays may also include any of the conventional propellants to deliver the material as a foam, in the case of a foam, or as a fine, uniform spray, in the case of an aerosol spray. Examples of suitable propellants include materials such as hydrofluorinated compounds, dichlorodifluoromethane, difluoroethane, dimethylether, isobutane, n-butane, propane, or trichloroflurometharie. A tonic or spray product having a low viscosity may also include an emulsifying agent. Examples of suitable emulsifying agents are anionic surfactants, cationic surfactants, nonionic surfactants, and mixtures thereof. Fluorosurfactants are suitable, particularly if the product is a spray composition and/or if it is a spray composition having a relatively low level of volatile organic solvents, such as alcohols, and relatively high levels of water (i.e., in excess of about 10%, by weight). If such an emulsifying agent is included, it is may be present at a level of from about 0.01 % to about 7.5% by weight of the composition. The level of propellant may be adjusted as desired, but is generally from about 3% to about 30% by weight of foam compositions and from about 15% to about 50% by weight of the aerosol spray compositions.

Suitable spray compositions include conventional, non-aerosol pump sprays, i.e., "atomizers," aerosol containers or cans having propellant, as described above, and also pump aerosol containers utilizing compressed air as the propellant.

A wide variety of additional components may be employed in the topical cosmetic/dermatologic compositions herein. The compositions disclosed herein may comprise a safe and effective amount of a pharmaceutical additive or adjuvant. The phrase "safe and effective" connotes an amount of an active agent high enough to significantly or positively modify the condition to be treated, but low enough to avoid serious side effects (at a reasonable benefit/risk ratio) within the scope of sound medical judgment. A safe and effective amount of the pharmaceutical active agent will vary with the specific active species, the ability of the composition to penetrate the active species through the skin, the amount of composition to be applied, the particular condition being treated, the age and physical condition of the patient being treated, the severity of the condition, the duration of the treatment, the nature of concurrent therapy, and like factors.

Suitable pharmaceutical active agents which may be used in the present compositions include antimicrobial drugs: antibacterials, antifungals, antiprotozoans, and antivirals. Antimicrobial drugs may comprise pharmaceutically acceptable salts of β-lactam drugs, amanfadine, amikacin, capreomycin, chlorhexidine, chlortetracycline, ciprofloxacin, clindamycin, doxycycline, erythromycin, ethambutol, gentamicin, kanamycin, lineomycin, methacycline, methenamine, metronidazole, miconazole, minocycline, neomycin, netilmicin, norfloxacin, oxytetracycline, paramomycin, pentamidine, quinolone drugs, streptomycin, tetracycline, tobramycin, and triclosan.

The subject cosmetic/dermatologic compositions may contain various emulsifiers when formulated as emulsions. These emulsifiers are useful for emulsifying the various carrier components of the compositions. Suitable emulsifiers may include any of a wide variety of nonionic, cationic, anionic, and zwitterionic emulsifiers known in the art.

Suitable emulsifier types include acyl lactylates, alkyl phosphates, carboxylic acid copolymers, esters and ethers of glucose, esters of glycerin, esters of propylene glycol, esters of sorbitan anhydrides, esters of sorbitol, ethoxylated ethers, ethoxylated alcohols, fatty acid amides, fatty acid esters of polyethylene glycol, fatty esters of polypropylene glycol, polyoxyethylene fatty ether phosphates, soaps, and mixtures thereof.

Suitable emulsifiers may include, but are not limited to, ceteareth-20, ceteth-10, cetyl phosphate, diethanolamine cetyl phosphate, glyceryl stearate, PEG-100 stearate, polyethylene glycol 20 sorbitan monolaurate, polyethylene glycol 5 soya sterol, polysorbate 60, polysorbate 80, potassium cetyl phosphate, PPG-2 methyl glucose ether distearate, steareth-20, and mixtures thereof. Suitable natural emulsifiers may include sucro esters, alkyl polyglycoside, monoglycerol esters, lecithin and its derivatives, or mixtures thereof.

The subject cosmetic/dermatologic compositions may also contain various emollients. Examples of suitable emollients include, but are not limited to, natural or naturally-derived oils such as vegetable oils, vegetable triglycerides, vegetable hydrocarbons, highly branched hydrocarbons, non-polar carboxylic acid and alcohol esters, volatile and non-volatile silicone oils, and mixtures thereof. For example, suitable emollients may include caprylic/capric triglyceride (CCT) and short chain $C_8$-$C_{14}$ vegetable-based hydrocarbons.

A variety of additional components may be incorporated into the subject cosmetic/dermatological compositions. Non-limiting examples of these additional components include cationic polymers and thickeners, chelators, gums and thickeners, low pH thickening agents, polymers for enhancing the film-forming properties and substantivity of the composition, sequestrants, humectants, skin penetrating aids, suspending agents, vitamins and derivatives thereof, preservatives and aesthetic components. Suitable additional components may include xanthan gum, diutan gum, guar gum, tara gum, cellulose gum and its derivatives, acrylates/$C_{10-30}$ alkyl acrylate, and acrylate-based crosspolymer and polyurethane-based polymers.

Suitable preservatives, which are conventional in the art and which prevent or retard microbial growth, and thus protect cosmetic products from spoilage, are set forth in CFTA International Cosmetic Ingredient Dictionary and Handbook, seventh edition, 2, 1654 (1997).

The cosmetic/dermatologic compositions of the present disclosure are administered in conventional fashion to provide the desired benefit. Such methods of use generally involve topical application of an effective amount of the composition onto the skin, which then is allowed to remain until absorbed into or removed from the skin.

While the compositions described above focus on fully-formulated compositions, it is contemplated that the micronized, non-solubilized waxes may also be used in concentrate formulations, which may then be added to other components to create a fully-formulated composition, such as a sunscreen composition. In such concentrate formulations, the micronized, non-solubilized waxes may be present in amounts of up to 80% (such as 75%, 70%, 65%, 60%, 55%, 50%, 45%, 40%, 35%, 30%, 25%, 20%, or 15%) by weight, based on the total weight of the concentrate formulation, with the remainder of the concentrate formulation including a diluent (e.g., oil and/or aqueous diluent), and optionally at least one of a dispersant, a stabilizer, or a thickener. The concentrate formulations may also include an active sun block agent, as well as other ingredients which may be suitable for use in fully-formulated compositions as described above. The concentrate formulations may also include other ingredients which may be useful for creating a desirable concentrate formulation (such as rheology modifiers, fillers, etc.), and which would not adversely affect the fully-formulated composition to which the concentrate is intended to be added.

## EXAMPLES

The subject matter disclosed herein is useful for improving sunscreen compositions, which may be better understood with reference to the following examples, which are set forth merely to further illustrate the subject matter disclose herein. The illustrative examples should not be construed as limiting the subject matter in any manner.

The examples shown below show the average in vitro SPF and water-resistance of different sunscreen formulations, demonstrating the effect of non-solubilized micronized natural waxes on promoting both properties. As shown below, it has been unexpectedly found that waxes in micronized form showed a significant improvement in SPF.

In the examples below, all parts and percentages are given by weight, unless otherwise indicated.

## In-Vitro SPF and Water Resistance Measurement Technique

The Labsphere UV-2000S Transmittance Analyzer was used to measure in-vitro SPF values of formulations on PMMA Plates. This instrument rapidly measures the diffuse transmittance of sunscreen samples in the ultraviolet wavelength region from 250 - 450 nm. Labsphere's Spectralon® integrating sphere incorporates a re-optimized xenon flash lamp to provide exceptional diffuse illumination of the product sample and minimize data integration time. UV-2000S Software was then utilized for UVA/UVB protection factors of sunscreen following a U.S. Food and Drug Administration (FDA) method. The critical wavelength was determined using the wavelength where 90% of the area under the curve lies, starting at 290 nm (CFR - Code of Federal Regulations Title 21).

Formulations were applied on polymethylmethacrylate (PMMA) sun plates suitable for UV transmittance measurements purchased from Helioscience. 1.1 mg/cm$^2$ of each formulation was applied onto a plate. For each formulation, 2 plates were made and, on each plate, 5 measurements were acquired at different locations. Each measurement is an average of 3 scans. Therefore, altogether, each SPF measurement is an average of 30 scans, providing a representative average of formulation spread over the entire PMMA plate to account for film thickness inconsistency and measurement error. As used herein, "standard deviation" (STD) refers to STD of all values measured for a particular sample, calculated using the following equation:

$$STD = \sqrt{\frac{\sum(x_i - \bar{x})^2}{n-1}}$$

wherein $x_i$ is a single measurement, $\bar{x}$ is the average of all measurements, and $n$ is the number of measurements taken. In the SPF measurements reported herein, 30 SPF measurements were taken for each sample.

The accuracy of the test depends upon the application of a precisely controlled amount of sunscreen product with a uniform distribution over the PMMA plate. Each formulation was first applied in a series of small dots over the entire PMMA plate using a positive displacement micropipette. The formulation was then spread evenly using a HelioScreen HD Spreadmaster robot. Each plate was then allowed to equilibrate for 30 minutes in the dark before pre-irradiation.

Photostability: Photostability of each formulation was evaluated by subjecting the formulation to a full spectrum light at 550 W/m$^2$ for 10 minutes, resembling day light exposure. Sample SPF value was then measured as described

above.

Water Resistance: Water resistance of each formulation was measured by immersing each plate in 30 $^{0}$C water for 80 min under slight turbulence at 115 RPM using a 708-DS Dissolution Apparatus purchased from Agilent Technologies. Each plate was then removed from water and allowed to dry and equilibrate for 30 minutes in the dark prior to measuring SPF value as described above.

Particle Size Analysis

The particle size, particle size distribution, and shape of the subject wax particles of the present disclosure may be evaluated by any known method, such as those described in US 2006/0292095 A1, for example, laser diffraction, ultrasonic extinction (acoustic spectroscopy), photo cross-correlation spectroscopy, granulometry, and/or image analysis (optical microscopy).

A suitable method of measuring particle size is a laser diffraction method. Laser diffraction methods use incident light scattering to measure particle size. A Mastersizer 3000 (Malvern Instruments Limited) laser diffraction apparatus was utilized to measure particle size values of certain non-solubilized micronized natural waxes described herein. According to the instrument's manufacturer (Malvern Instruments Limited: www.malvern.com), the Mastersizer 3000 uses the technique of laser diffraction to measure the size of particles. It does this by measuring the intensity of light scattered as a laser beam passes through a dispersed particulate sample. When a beam of light is partly blocked by an obstacle, some of the light is scattered around the object, and light and dark bands are often seen at the edge of the shadow; this effect is known as diffraction. These effects can be modelled using the Huygens-Fresnel principle. Huygens postulated that every point on a primary wave front acts as a source of spherical secondary wavelets and the sum of these secondary wavelets determines the form of the wave at any subsequent time. Fresnel developed an equation using the Huygens wavelets together with the principle of superposition of waves, which models these diffraction effects quite well. The angle of the laser beam and particle size have an inversely proportional relationship, where the laser beam angle increases as particle size decreases and vice versa. Using the instrument's software, this data is then analyzed to calculate the size of the particles that created the scattering pattern. A neat powder or a dispersed sample passes though the measurement area of the optical bench, where a laser beam illuminates the particles. Samples were measured: (i) as solid powder, using Aero-S accessory for solid samples with a gap of 2.0 to 4.0 mm; or (ii) in liquid dispersion, using Hydro SV accessory for liquid samples. In the latter instance, the powdered waxes were pre-dispersed at 5% in Caprylic/Capric Triglyceride (CCT) containing 2% Matrifuse S-1, and measured before and after melting at 90 °C and cooling down to 25 °C. Measurements using Hydro SV were done in Caprylic/Capric Triglyceride (CCT) media at 1000 speed (arbitrary units), adding droplets of sample until the obscuration was in the measurement range. A series of detectors then accurately measures the intensity of light scattered by the particles within the sample for both red and blue light wavelengths and over a wide range of angles. The Mastersizer 3000 software controls the system during the measurement process and analyzes the scattering data to calculate a particle size in the form of D10, D50 and D90 volume average particle size distributions. (For simplicity, these particle size distributions are referred to as "D10 particle size", "D50 particle size", and "D90 particle size" herein.) It also provides both instant feedback during method development and expert advice on the quality of the results.

D10, D50 and D90 of particle size distribution is reported for monodisperse and polydisperse particle size. FIG. 3 shows a chart of an illustrative particle size distribution and percentiles (D10, D50 and D90), which are the size below which there is a certain volume of the sample. For example, D50 indicates that 50 volume percent of the particles are below the reported value. Using the methods described herein, or other methods known in the art, it is possible to obtain various measurements of particle size distribution, such as D1, D3, D5, D10, D50, D90, D95, D97 and D99.

Effect of Temperature on Particle Size/Shape

When micronized natural waxes were heated above their melting point (such as 80 to 90 °C), particle size and shape significantly change, and these samples did not show the SPF and water resistance properties shown in samples of non-solubilized, micronized waxes. In light of this, it is even more unexpected that both water resistance and SPF were improved by using the non-solubilized, micronized waxes at temperatures below the melting point of the waxes. While not wishing to be bound to or by any particular theory or principle, it is believed that the subject non-solubilized, micronized natural waxes provide water resistance by forming a hydrophobic film on the surface of a substrate. In addition, it is believed that the subject non-solubilized, micronized, natural waxes enhance the efficacy of the sunscreen formulation by diffracting incoming light and increasing the likelihood of the incident light encountering a sunscreen molecule.

In the following examples, shown in Table 1, the identified waxes were supplied in powder form, then dispersed in caprylic/capric triglyceride (CCT), followed by heating to melt the wax. In some of the Examples presented herein, different grades of carnauba wax were used. Carnauba wax is commercially available in three grades, which are known to those of ordinary skill in the art: T1, T3 and T4, which vary in their purity and color, with lower grades being darker in color. The dispersions contained 93 weight percent CCT, 5 weight percent of the wax, and 2 weight percent of a hyperdispersant,

based on the total weight of the dispersion. "OPS" is the original D50 particle size, in μm. "1 h" refers to the D50 particle size, in μm, after heating the dispersion to 90 °C for one hour. "24 h" refers to the D50 particle size, in μm, after heating the dispersion to 90 °C for 24 hours. Particle size values were measured using the laser diffraction method described above.

Table 1

| Example | Wax | Form | OPS | 1 h | 24 h |
|---|---|---|---|---|---|
| 1 | Carnauba T3 | Powder | 6.1 | N/A | N/A |
| 2 | Carnauba T3 | 5 % Dispersion in CCT | 7.9 | 86.0 | 93.5 |
| 3 | Castor | Powder | 24.9 | N/A | N/A |
| 4 | Castor | 5 % Dispersion in CCT | 21.7 | 149 | 124 |
| 5 | Rice Bran A | Powder | 9.18 | N/A | N/A |
| 6 | Rice Bran A | 5 % Dispersion in CCT | 10.7 | 62.6 | 63.5 |
| 7 | Rice Bran B | Powder | 8.9 | N/A | N/A |
| 8 | Rice Bran B | 5 % Dispersion in CCT | 9.1 | 29.3 | 33.6 |
| 9 | Synthetic | Powder | 10.1 | N/A | N/A |
| 10 | Synthetic | 5 % Dispersion in CCT | 10.1 | 80.1 | 90.1 |

Examples 11 through 15 were prepared with the ingredients and amounts listed in Table 2, below. As used herein, "INCI" refers to the International Nomenclature of Cosmetic Ingredients system of naming cosmetic ingredients.

Table 2

| Ingredient INCI Name | Wt. % | Function |
|---|---|---|
| SPF Booster (carnauba wax) | 5.0 | SPF Booster |
| Deionized Water | QS To 100.0 | Diluent |
| Disodium EDTA | 0.1 | Chelating Agent |
| Acrylates/$C_{10-30}$ Alkyl Acrylate Crosspolymer (Pemulen™ EZ-4U Polymeric Emulsifier) | 0.15 | Polymeric Emulsifier /Rheology Modifier |
| 1,3 Propanediol | 10.0 | Humectant |
| Octocrylene | 10.0 | UV Filter |
| Homosalate | 10.0 | UV Filter |
| Ethylhexyl Salicylate | 5.0 | UV Filter |
| Butyl Methoxydibenzoyl Methane | 3.0 | UV Filter |
| Diisopropyl Sebacate (Schercemol™ DIS ester) | 6.0 | Emollient |
| Polyglyceryl-3 Laurate (Hydramol™ TGL ester) | 0.5 | Emulsifier/Humectant |
| Sodium Hydroxide (18 wt%) | QS to pH=5-6 | pH Adjusting Agent |
| Phenoxyethanol (and) Ethylhexylglycerin | 0.5 | Preservative |

The compositions described in Table 2 were prepared as follows: The compositions described above were prepared using a typical emulsion preparation method. Water and chelating agent (Disodium EDTA) were added to an appropriately sized vessel and mixed using a marine blade at 400 RPM until chelating agent was completely dissolved. Rheology modifier (Pemulen™ EZ-4U) was then added and mixed at 400 RPM at 50 °C until it was uniformly dispersed. 1,3 propanediol was then added to the water phase. In a separate appropriately sized vessel, oil phase ingredients (octocrylene, homosalate, ethylhexyl salicylate, butyl methoxydibenzoyl methane, diisopropyl sebacate, polyglyceryl-3 Laurate) were added and mixed at 70 °C using a marine blade at 400 RPM until a one-phase homogenous blend was achieved. Oil phase was then cooled down to 50 °C. The SPF Booster was then added to the appropriate phase and mixed until uniformly dispersed. Both oil and water phases were then equilibrated at 50 °C. Oil phase was then added to water phase and mixed. Neutralizer was then added which results in significant increase in viscosity as rheology modifier forms

its structure in the emulsion. Therefore, an increase in mixing speed may be required. Preservative was then added, and the formulation was allowed to cool while mixing for another 30 min.

Example 11 was prepared at a processing temperature of 50 °C. Example 12 was prepared at a processing temperature of 60 °C. Example 13 was prepared at a processing temperature of 70 °C. Example 14 was prepared at a processing temperature of 80 °C. Example 15 was prepared at a processing temperature of 90 °C. All of Examples 11 through 15 used carnauba wax as the SPF booster. The melting point of carnauba wax is 82 °C. Table 3 reports the Sun Protection Factor (SPF), the standard deviation (STD) of the SPF measurements, and the percent increase or decrease (%) of each composition as compared to a control composition having no SPF booster.

Table 3

| Example | SPF | STD | % |
|---|---|---|---|
| 11 | 101.4 | 38.2 | 147 |
| 12 | 111.5 | 28.1 | 171 |
| 13 | 95.1 | 38.4 | 131 |
| 14 | 66.2 | 31.8 | 61 |
| 15 | 40.3 | 14.0 | -2 |

These results show that the non-solubilized, micronized waxes described herein provide SPF boosting when in the non-solubilized, micronized form (Examples 11-14), as compared to a solubilized wax (Example 15). Example 15 is considered to include a solubilized wax, as the wax was melted during preparation of the composition of Example 15. Also, performance of Example 14 was not quite as good as shown with Examples 11 through 13; without wishing to be limited by theory, it is believed that this is a result of Example 14 being processed at a temperature close to, but still less than, the melting point of the SPF booster (carnauba wax, with a melting point of 82 °C), which may have begun to alter the wax particles. However, Example 14 is considered to include a non-solubilized, micronized wax, since the wax was not heated in excess of its melting point.

Examples 16 through 37 were prepared with the ingredients and amounts listed in Table 4, below.

Table 4

| Ingredient INCI Name | Wt. % | Function |
|---|---|---|
| SPF Booster | 5.0 | SPF Booster |
| Deionized Water | QS To 100.0 | Diluent |
| Disodium EDTA | 0.1 | Chelating Agent |
| Acrylates/$C_{10-30}$ Alkyl Acrylate Crosspolymer (Pemulen™ EZ-4U Polymeric Emulsifier) | 0.15 | Polymeric Emulsifier /Rheology Modifier |
| 1,3 Propanediol | 10.0 | Humectant |
| Octocrylene | 10.0 | UV Filter |
| Homosalate | 10.0 | UV Filter |
| Ethylhexyl Salicylate | 5.0 | UV Filter |
| Butyl Methoxydibenzoyl Methane | 3.0 | UV Filter |
| Diisopropyl Sebacate (Schercemol™ DIS ester) | 6.0 | Emollient |
| Polyglyceryl-3 Laurate (Hydramol™ TGL ester) | 0.5 | Emulsifier/Humectant |
| Sodium Hydroxide (18 wt%) | QS to pH=5-6 | pH Adjusting Agent |
| Phenoxyethanol (and) Ethylhexylglycerin | 0.5 | Preservative |

The compositions described in Table 4 were prepared according to the procedure described above with regard to the compositions described in Table 2.

All of Examples 16 through 37 were prepared at a processing temperature of 50 °C. The SPF booster used in each example is listed in Table 5. Table 5 also lists the phase of the composition to which the SPF booster was added, the Sun Protection Factor (SPF), the standard deviation (STD) of the SPF measurements, the percent increase or decrease

(%) of each composition as compared to the control composition (Example 16) having no SPF booster, and the critical wavelength (CWL), in nm, of each composition. With regard to Examples 35 and 37, the compositions could not be processed because the styrene/acrylate copolymers could only be processed in the water phase.

Table 5

| EX# | SPF Booster | Phase | SPF | STD | % | CWL |
|---|---|---|---|---|---|---|
| 16 | None | N/A | 41.1 | 10.6 | N/A | 377.1 |
| 17 | Carnauba T3 | Oil Phase | 67.3 | 45.9 | 64 | 376.9 |
| 18 | Carnauba T3 | Water Phase | 84.9 | 21.5 | 107 | 377.0 |
| 19 | Carnauba | Post Add | 102.1 | 20.3 | 149 | 377.0 |
| 20 | Castor | Oil Phase | 31.3 | 16.2 | -24 | 377.7 |
| 21 | Castor | Water Phase | 127.2 | 16.2 | 210 | 377.0 |
| 22 | Castor | Post Add | 156.0 | 13.7 | 280 | 376.0 |
| 23 | Synthetic | Oil Phase | 83.4 | 30.6 | 103 | 376.5 |
| 24 | Synthetic | Water Phase | 239.7 | 31.4 | 484 | 376.4 |
| 25 | Synthetic | Post Add | 96.3 | 12.7 | 134 | 377.0 |
| 26 | Rice Bran A | Oil Phase | 101.4 | 33.7 | 147 | 376.6 |
| 27 | Rice Bran A | Water Phase | 75.4 | 38.6 | 84 | 377.1 |
| 28 | Rice Bran A | Post Add | 48.6 | 23.7 | 18 | 377.1 |
| 29 | Rice Bran B | Oil Phase | 48.7 | 17.4 | 19 | 377.0 |
| 30 | Rice Bran B | Water Phase | 81.4 | 79.6 | 98 | 377.0 |
| 31 | Rice Bran B | Post Add | 86.7 | 46.4 | 111 | 377.0 |
| 32 | Sunflower | Oil Phase | 102.3 | 25.7 | 149 | 377.0 |
| 33 | Sunflower | Water Phase | 73.6 | 8.6 | 79 | 378.0 |
| 34 | Sunflower | Post Add | 106.1 | 11.5 | 158 | 377.0 |
| 35 | Styrene/Acrylate Copolymer | Oil Phase | N/A | | | |
| 36 | Styrene/Acrylate Copolymer | Water Phase | 97.3 | 37.9 | 137 | 377.8 |
| 37 | Styrene/Acrylate Copolymer | Post Add | N/A | | | |

In addition to showing the benefits of the non-solubilized, micronized waxes with regard to SPF boosting, these examples also show that the non-solubilized, micronized waxes may be added to any phase of the compositions, which had not been previously discovered.

Examples 38 through 56 were prepared with the ingredients and amounts listed in Table 6, below.

Table 6

| Ingredient INCI Name | Wt. % | Function |
|---|---|---|
| SPF Booster | 1 or 3 or 5.0 | SPF Booster |
| Deionized Water | QS To 100.0 | Diluent |
| Disodium EDTA | 0.1 | Chelating Agent |
| Acrylates/$C_{10-30}$ Alkyl Acrylate Crosspolymer (Pemulen™ EZ-4U Polymeric Emulsifier) | 0.15 | Polymeric Emulsifier /Rheology Modifier |
| 1,3 Propanediol | 10.0 | Humectant |
| Octocrylene | 10.0 | UV Filter |
| Homosalate | 10.0 | UV Filter |

(continued)

| Ingredient INCI Name | Wt. % | Function |
|---|---|---|
| Ethylhexyl Salicylate | 5.0 | UV Filter |
| Butyl Methoxydibenzoyl Methane | 3.0 | UV Filter |
| Diisopropyl Sebacate (Schercemol™ DIS ester) | 6.0 | Emollient |
| Polyglyceryl-3 Laurate (Hydramol™ TGL ester) | 0.5 | Emulsifier/Humectant |
| Sodium Hydroxide (18 wt. %) | QS to pH=5-6 | pH Adjusting Agent |
| Phenoxyethanol (and) Ethylhexylglycerin | 0.5 | Preservative |

The compositions described in Table 6 were prepared according to the procedure described above with regard to the compositions described in Table 2.

All of Examples 38 through 56 were prepared at a processing temperature of 50 °C. The SPF booster used in each example, as well as the amount of SPF booster in each example, is listed in Table 7. The SPF boosters shown in Table 7 were all added to the compositions "Post Add". Table 7 also lists the standard deviation (STD) of the SPF measurements, the percent increase or decrease (%) of each composition as compared to the control composition (Example 38) having no SPF booster, and the critical wavelength (CWL), in nm, of each composition.

Table 7

| EX# | SPF Booster | wt. % | SPF | STD | % | CWL |
|---|---|---|---|---|---|---|
| 38 | N/A | 0 | 41.1 | 10.6 | N/A | 377.1 |
| 39 | Carnauba T3 | 5 | 102.1 | 20.3 | 149 | 377.0 |
| 40 | Carnauba T3 | 3 | 104.8 | 16.9 | 155 | 377.0 |
| 41 | Carnauba T3 | 1 | 83.7 | 14.3 | 104 | 377.0 |
| 42 | Castor | 5 | 156.0 | 13.7 | 280 | 376.0 |
| 43 | Castor | 3 | 74.3 | 14.3 | 81 | 376.0 |
| 44 | Castor | 1 | 62.7 | 14.2 | 53 | 377.1 |
| 45 | Synthetic | 5 | 96.3 | 12.7 | 134 | 377.0 |
| 46 | Synthetic | 3 | 76.3 | 15.3 | 86 | 376.1 |
| 47 | Synthetic | 1 | 73.8 | 10.1 | 80 | 376.6 |
| 48 | Rice Bran A | 5 | 48.6 | 23.7 | 18 | 377.1 |
| 49 | Rice Bran A | 3 | 98.7 | 10.6 | 140 | 376.6 |
| 50 | Rice Bran A | 1 | 48.3 | 5.1 | 18 | 376.8 |
| 51 | Rice Bran B | 5 | 86.7 | 46.4 | 111 | 377.0 |
| 52 | Rice Bran B | 3 | 91.0 | 12.7 | 122 | 377.0 |
| 53 | Rice Bran B | 1 | 56.1 | 10.1 | 37 | 377.1 |
| 54 | Sunflower | 5 | 106.1 | 11.5 | 158 | 377.0 |
| 55 | Sunflower | 3 | 64.3 | 9.6 | 57 | 376.8 |
| 56 | Sunflower | 1 | 53.9 | 5.5 | 31 | 376.7 |

Examples 57 through 76 were prepared with the ingredients and amounts listed in Table 8, below.

Table 8

| Ingredient INCI Name | Wt. % | Function |
|---|---|---|
| SPF Booster | 1 or 3 or 5.0 | SPF Booster |

(continued)

| Ingredient INCI Name | Wt. % | Function |
|---|---|---|
| Deionized Water | QS To 100.0 | Diluent |
| Disodium EDTA | 0.1 | Chelating Agent |
| Acrylates/$C_{10-30}$ Alkyl Acrylate Crosspolymer | 0.15 | Polymeric Emulsifier /Rheology Modifier |
| 1,3 Propanediol | 10.0 | Humectant |
| Octocrylene | 10.0 | UV Filter |
| Homosalate | 10.0 | UV Filter |
| Ethylhexyl Salicylate | 5.0 | UV Filter |
| Butyl Methoxydibenzoyl Methane | 3.0 | UV Filter |
| Diisopropyl Sebacate (Schercemol™ DIS ester) | 6.0 | Emollient |
| Polyglyceryl-3 Laurate (Hydramol™ TGL ester) | 0.5 | Emulsifier/Humectant |
| Sodium Hydroxide (18 wt%) | QS to pH=5-6 | pH Adjusting Agent |
| Phenoxyethanol (and) Ethylhexylglycerin | 0.5 | Preservative |

The compositions described in Table 8 were prepared according to the procedure described above with regard to the compositions described in Table 2.

All of Examples 57 through 76 were prepared at a processing temperature of 50 °C. The SPF booster used in each example, as well as the amount ("A", in wt.%) of SPF booster in each example, is listed in Table 9. Table 9 also lists the phase (P) of the composition to which the SPF booster was added (O=oil phase; W=water phase; PA = "post add"), the Sun Protection Factor (SPF), the post-photostability SPF (P-SPF), the post-water resistance SPF (W-SPF), and the standard deviation (STD) of each of the SPF measurements. "SA" refers to styrene/acrylate copolymer.

Table 9

| EX# | SPF Booster | A | P | SPF | STD | P-SPF | STD | W-SPF | STD |
|---|---|---|---|---|---|---|---|---|---|
| 57 | N/A | 0 | N/A | 41.1 | 10.6 | 42.8 | 9.5 | 37.4 | 9.1 |
| 58 | Carnauba T3 | 5 | O | 67.3 | 45.9 | 64.8 | 44.2 | 89.4 | 76.7 |
| 59 | Carnauba T3 | 3 | PA | 104.8 | 16.9 | 103.0 | 15.3 | 106.2 | 17.6 |
| 60 | Carnauba T3 | 1 | PA | 83.7 | 14.3 | 78.4 | 12.0 | 74.6 | 8.7 |
| 61 | Castor | 5 | O | 31.3 | 16.2 | 35.3 | 15.7 | 33.0 | 15.2 |
| 62 | Castor | 3 | PA | 74.3 | 14.3 | 73.1 | 14.5 | 62.4 | 10.4 |
| 63 | Castor | 1 | PA | 62.7 | 14.2 | 60.8 | 14.3 | 55.0 | 12.6 |
| 64 | Synthetic | 5 | O | 83.4 | 30.6 | 79.6 | 29.8 | 80.4 | 26.3 |
| 65 | Synthetic | 3 | PA | 76.3 | 15.3 | 67.7 | 10.5 | 64.7 | 7.0 |
| 66 | Synthetic | 1 | PA | 73.8 | 10.1 | 60.8 | 7.9 | 56.4 | 7.3 |
| 67 | Rice Bran A | 5 | O | 101.4 | 33.7 | 97.8 | 32.7 | 145.9 | 51.2 |
| 68 | Rice Bran A | 3 | PA | 98.7 | 10.6 | 93.9 | 10.6 | 135.04 | 13.5 |
| 69 | Rice Bran A | 1 | PA | 48.3 | 5.1 | 48.0 | 5.3 | 44.4 | 4.8 |
| 70 | Rice Bran B | 5 | O | 48.7 | 31.7 | 50.3 | 32.8 | 55.6 | 35.2 |
| 71 | Rice Bran B | 3 | PA | 91.0 | 12.7 | 86.4 | 12.3 | 86.3 | 12.8 |
| 72 | Rice Bran B | 1 | PA | 56.1 | 10.1 | 56.0 | 9.3 | 54.1 | 7.9 |
| 73 | Sunflower | 5 | O | 102.3 | 25.7 | 105.1 | 24.7 | 108.0 | 25.1 |
| 74 | Sunflower | 3 | PA | 64.3 | 9.6 | 65.4 | 9.8 | 54.8 | 5.6 |
| 75 | Sunflower | 1 | PA | 53.9 | 5.5 | 52.2 | 5.0 | 44.7 | 3.8 |

(continued)

| EX# | SPF Booster | A | P | SPF | STD | P-SPF | STD | W-SPF | STD |
|---|---|---|---|---|---|---|---|---|---|
| 76 | Styrene/Acrylate Copolymer | 5 | W | 97.3 | 37.9 | 108.7 | 22.6 | 69.7 | 35.5 |

Examples 77 through 86 were prepared with the ingredients and amounts listed in Table 10, below.

Table 10

| Ingredient INCI Name | Wt. % | Function |
|---|---|---|
| SPF Booster 1 | 2.5 | SPF Booster |
| SPF Booster 2 | 2.5 | SPF Booster |
| Deionized Water | QS To 100.0 | Diluent |
| Disodium EDTA | 0.1 | Chelating Agent |
| Acrylates/$C_{10-30}$ Alkyl Acrylate Crosspolymer (Pemulen™ EZ-4U Polymeric Emulsifier) | 0.15 | Polymeric Emulsifier /Rheology Modifier |
| 1,3 Propanediol | 10.0 | Humectant |
| Octocrylene | 10.0 | UV Filter |
| Homosalate | 10.0 | UV Filter |
| Ethylhexyl Salicylate | 5.0 | UV Filter |
| Butyl Methoxydibenzoyl Methane | 3.0 | UV Filter |
| Diisopropyl Sebacate (Schercemol™ DIS ester) | 6.0 | Emollient |
| Polyglyceryl-3 Laurate (Hydramol™ TGL ester) | 0.5 | Emulsifier/Humectant |
| Sodium Hydroxide (18 wt%) | QS to pH=5-6 | pH Adjusting Agent |
| Phenoxyethanol (and) Ethylhexylglycerin | 0.5 | Preservative |

The compositions described in Table 10 were prepared according to the procedure described above with regard to the compositions described in Table 2.

All of Examples 77 through 86 were prepared at a processing temperature of 50 °C. The SPF boosters ("Booster 1" and "Booster 2") used in each example are listed in Table 11. All of the waxes of Examples 77 through 86 were "post added" to the compositions. Table 11 also lists the Sun Protection Factor (SPF), the post-photostability SPF (P-SPF), the post-water resistance SPF (W-SPF), and the standard deviation (STD) of each of the SPF measurements.

Table 11

| EX# | Booster 1 | Booster 2 | SPF | STD | P-SPF | STD | W-SPF | STD |
|---|---|---|---|---|---|---|---|---|
| 77 | N/A | N/A | 41.1 | 10.6 | 42.8 | 9.5 | 37.4 | 9.1 |
| 78 | Carnauba T3 | Rice Bran A | 110.7 | 25.5 | 105.1 | 17.8 | 153.7 | 32.9 |
| 79 | Carnauba T3 | Rice Bran B | 108.0 | 42.6 | 102.5 | 40.4 | 111.8 | 42.2 |
| 80 | Carnauba T3 | Castor | 34.5 | 13.5 | 33.3 | 12.2 | 34.1 | 13.8 |
| 81 | Rice Bran A | Rice Bran B | 102.8 | 24.6 | 99.6 | 25.5 | 125.9 | 35.2 |
| 82 | Rice Bran A | Castor | 24.5 | 10.5 | 24.0 | 9.9 | 26.4 | 11.1 |
| 83 | Rice Bran B | Castor | 84.4 | 40.0 | 79.8 | 34.5 | 83.6 | 38.8 |
| 84 | Carnauba T1 | Rice Bran A | 85.0 | 2.2 | 80.5 | 4.8 | 105.2 | 7.4 |
| 85 | Carnauba T1 | Rice Bran B | 94.7 | 9.4 | 93.8 | 7.4 | 107.2 | 8.4 |
| 86 | Carnauba T1 | Castor | 104.1 | 21.6 | 108.1 | 19.0 | 111.6 | 20.6 |

Examples 87 through 93 were prepared with the ingredients and amounts listed in Table 12, below.

Table 12

| Ingredient INCI Name | Wt. % | Function |
|---|---|---|
| SPF Booster | 5.0 | SPF Booster |
| Deionized Water | QS To 100.0 | Diluent |
| Disodium EDTA | 0.1 | Chelating Agent |
| Acrylates/$C_{10-30}$ Alkyl Acrylate Crosspolymer (Pemulen™ EZ-4U Polymeric Emulsifier) | 0.15 | Polymeric Emulsifier /Rheology Modifier |
| 1,3 Propanediol | 10.0 | Humectant |
| Octocrylene | 10.0 | UV Filter |
| Homosalate | 10.0 | UV Filter |
| Ethylhexyl Salicylate | 5.0 | UV Filter |
| Butyl Methoxydibenzoyl Methane | 3.0 | UV Filter |
| Diisopropyl Sebacate (Schercemol™ DIS ester) | 6.0 | Emollient |
| Polyglyceryl-3 Laurate (Hydramol™ TGL ester) | 0.5 | Emulsifier/Humectant |
| Sodium Hydroxide (18 wt%) | QS to pH=5-6 | pH Adjusting Agent |
| Phenoxyethanol (and) Ethylhexylglycerin | 0.5 | Preservative |

The compositions described in Table 12 were prepared according to the procedure described above with regard to the compositions described in Table 2.

All of Examples 87 through 93 were prepared at a processing temperature of 50 °C, and included carnauba wax as the SPF booster. All of the waxes of Examples 87 through 93 were "post added" to the compositions, except for Example 88, which was added to the oil phase. Table 13 lists the amount ("A", in wt.%) and grade ("G") of carnauba wax used, the Sun Protection Factor (SPF), the post-photostability SPF (P-SPF), the post-water resistance SPF (W-SPF), and the standard deviation (STD) of each of the SPF measurements.

Table 13

| EX# | A | G | SPF | STD | P-SPF | STD | W-SPF | STD |
|---|---|---|---|---|---|---|---|---|
| 87 | 0 | N/A | 41.1 | 10.6 | 42.8 | 9.5 | 37.4 | 9.1 |
| 88 | 5 | T3 | 67.3 | 45.9 | 64.8 | 44.2 | 89.4 | 76.7 |
| 89 | 3 | T3 | 104.8 | 16.9 | 103.0 | 15.3 | 106.2 | 17.6 |
| 90 | 1 | T3 | 83.7 | 14.3 | 78.4 | 12.0 | 74.6 | 8.7 |
| 91 | 5 | T1 | 79.8 | 12.3 | 76.5 | 10.3 | 80.7 | 10.6 |
| 92 | 3 | T1 | 79.0 | 6.7 | 75.8 | 5.6 | 76.3 | 3.2 |
| 93 | 1 | T1 | 74.0 | 6.03 | 70.4 | 5.1 | 56.3 | 3.3 |

Examples 94 and 95 are provided to show that certain of the subject non-solubilized, micronized waxes do not act as sun block agents/UV filters. Examples 94 and 95 were prepared with the ingredients and amounts listed in Table 14, below.

Table 14

| Ingredient INCI Name | Wt. % | Function |
|---|---|---|
| SPF Booster | 5.0 | SPF Booster |
| Deionized Water | QS To 100.0 | Diluent |
| Disodium EDTA | 0.1 | Chelating Agent |
| Acrylates/$C_{10-30}$ Alkyl Acrylate Crosspolymer (Pemulen™ EZ-4U Polymeric Emulsifier) | 0.15 | Polymeric Emulsifier /Rheology Modifier |

(continued)

| Ingredient INCI Name | Wt. % | Function |
|---|---|---|
| 1,3 Propanediol | 0.0 | Humectant |
| Octocrylene | 0.0 | UV Filter |
| Homosalate | 0.0 | UV Filter |
| Ethylhexyl Salicylate | 0.0 | UV Filter |
| Butyl Methoxydibenzoyl Methane | 0.0 | UV Filter |
| Diisopropyl Sebacate (Schercemol™ DIS ester) | 6.0 | Emollient |
| Polyglyceryl-3 Laurate (Hydramol™ TGL ester) | 0.5 | Emulsifier/Humectant |
| Sodium Hydroxide (18 wt%) | QS to pH=5-6 | pH Adjusting Agent |
| Phenoxyethanol (and) Ethylhexylglycerin | 0.5 | Preservative |

The compositions described in Table 14 were prepared according to the procedure described above with regard to the compositions described in Table 2.

Examples 94 and 95 were prepared at a processing temperature of 50 °C. The waxes of Examples 94 and 95 were "post added" to the compositions. Table 15 lists the SPF booster used in each example, the Sun Protection Factor (SPF) and the standard deviation (STD) of each of the SPF measurements.

Table 15

| EX# | SPF Booster | SPF | STD |
|---|---|---|---|
| 94 | Carnauba | 1.3 | <0.1 |
| 95 | Rice Bran A | 1.0 | <0.1 |

Examples 96 through 111 were prepared with the ingredients and amounts listed in Table 16, below.

Table 16

| Ingredient INCI Name | Wt. % | Function |
|---|---|---|
| SPF Booster | 5.0 | SPF Booster |
| Deionized Water | QS To 100.0 | Diluent |
| Neopentyl Glycol Diethylhexanoate (Schercemol™ NGDO) | 5.0 | Emollient |
| Cetyl Octanoate (Schercemol™ CO Ester) | 15.0 | Emollient/Sensory Modifier |
| Isopropyl Isostearate (Schercemol™ 318 Ester) | 10.0 | Emollient/Sensory Modifier |
| Polyhydroxystearic Acid and Neopentyl Glycol Diethylhexanoate (Matrifuse™ S-1 Dispersant) | 1.0 | Dispersant/Stabilizer |
| Zinc Oxide (and) Triethoxycaprylylsilane | 10.0 | UV Filter |
| Titanium Dioxide (and) Aluminum Hydroxide (and) Stearic Acid | 5.0 | UV Filter |
| Polyurethane-62 (and) Trideceth-6 (Avalure™ Flex-6 Polymer) | 0.5 | Polymeric Emulsifier/ Film Former |
| Sodium Chloride | 0.7 | Stabilizer |
| Phenoxyethanol (and) Ethylhexylglycerin | 0.5 | Preservative |

The compositions described in Table 16 were prepared as follows: In an appropriately sized vessel, mineral UV filters (zinc oxide and titanium oxide) were homogenized in emollients (neopentyl glycol diethylhexanoate, cetyl octanoate, isopropyl isostearate) using an Ultra-Turrax® (IKA) homogenizer at 5000 RPM for 15 min at RT. Homogenization step is frequently used when dispersing mineral UV filters to break them down to their primary particle size. Stabilizer/hyperdispersant (polyhydroxystearic acid and neopentyl glycol diethylhexanoate) was then added to dispersed

mineral UV filters and the mixture was then heated to about 50 °C while stirring using a dispersion blade at 200 RPM. In a separate appropriately size vessel, water phase (water, rheology modifier (polyurethane-62 and trideceth-6), sodium chloride) were heated to about 50 °C and mixed using a dispersion blade at 200 RPM until rheology modifier was uniformly dispersed. The SPF Booster was then added to the appropriate phase and mixed until uniformly dispersed. Once both oil and water phases reach the desired temperatures and are homogenous, water phase was slowly added to oil phase while mixing using a dispersion blade at 200 RPM. Resulting emulsion was then cooled to room temperature and preservative (phenoxyethanol (and) Ethylhexylglycerin) was then added.

All of Examples 96 through 111 were prepared at a processing temperature of 50 °C. The SPF booster used in each example is listed in Table 17. Table 17 also lists the phase ("P") in which the SPF booster was added, the SPF of each composition, the standard deviation (STD) of the SPF measurements, the percent increase or decrease (%) of each composition as compared to the control composition (Example 96) having no SPF booster, and the critical wavelength (CWL), in nm, of each composition. With regard to Examples 109 and 111, the compositions could not be processed because the styrene/acrylate copolymers could only be processed in the water phase.

Table 17

| EX# | SPF Booster | P | SPF | STD | % | CWL |
|---|---|---|---|---|---|---|
| 96 | N/A | N/A | 17.9 | 5.8 | N/A | 371.0 |
| 97 | Carnauba T3 | Oil Phase | 29.7 | 8.2 | 66 | 370.9 |
| 98 | Carnauba T3 | Water Phase | 26.9 | 7.2 | 50 | 370.9 |
| 99 | Carnauba T3 | Post Add | 30.3 | 8.6 | 69 | 369.8 |
| 100 | Synthetic | Oil Phase | 36.5 | 8.9 | 104 | 370.0 |
| 101 | Synthetic | Water Phase | 32.2 | 11.9 | 80 | 370.1 |
| 102 | Synthetic | Post Add | 25.6 | 10.0 | 43 | 370.1 |
| 103 | Rice Bran A | Oil Phase | 22.7 | 9.3 | 27 | 370.8 |
| 104 | Rice Bran A | Water Phase | 23.5 | 7.4 | 31 | 371.0 |
| 105 | Rice Bran A | Post Add | 27.2 | 8.1 | 52 | 370.5 |
| 106 | Rice Bran B | Oil Phase | 32.5 | 9.1 | 82 | 370.4 |
| 107 | Rice Bran B | Water Phase | 31.2 | 9.2 | 74 | 371.0 |
| 108 | Rice Bran B | Post Add | 24.8 | 5.3 | 38 | 370.4 |
| 109 | Styrene/Acrylate Copolymer | Oil Phase | N/A, only processable in water phase | | | |
| 110 | Styrene/Acrylate Copolymer | Water Phase | 38.5 | 15.0 | 102 | 371.5 |
| 111 | Styrene/Acrylate Copolymer | Post Add | N/A, only processable in water phase | | | |

Examples 112 through 124 were prepared with the ingredients and amounts listed in Table 18, below.

Table 18

| Ingredient INCI Name | Wt. % | Function |
|---|---|---|
| SPF Booster | 5.0 | SPF Booster |
| Deionized Water | QS To 100.0 | Diluent |
| Neopentyl Glycol Diethylhexanoate (Schercemol™ NGDO) | 5.0 | Emollient |
| Cetyl Octanoate (Schercemol™ CO Ester) | 15.0 | Emollient/Sensory Modifier |
| Isopropyl Isostearate (Schercemol™ 318 Ester) | 10.0 | Emollient/Sensory Modifier |
| Polyhydroxystearic Acid and Neopentyl Glycol Diethylhexanoate (Matrifuse™ S-1 Dispersant) | 1.0 | Dispersant/Stabilizer |
| Zinc Oxide (and) Triethoxycaprylylsilane | 10.0 | UV Filter |
| Titanium Dioxide (and) Aluminum Hydroxide (and) Stearic Acid | 5.0 | UV Filter |

(continued)

| Ingredient INCI Name | Wt. % | Function |
|---|---|---|
| Polyurethane-62 (and) Trideceth-6 (Avalure™ Flex-6 Polymer) | 0.5 | Polymeric Emulsifier/ Film Former |
| Sodium Chloride | 0.7 | Stabilizer |
| Phenoxyethanol (and) Ethylhexylglycerin | 0.5 | Preservative |

The compositions described in Table 18 were prepared according to the procedure described above with regard to the compositions described in Table 16.

All of Examples 112 through 124 were prepared at a processing temperature of 50 °C, and the SPF booster was "post added" to the composition. The SPF booster used in each example, as well as the amount ("A", in wt.%) of SPF booster in each example, is listed in Table 19. Table 19 also lists the the SPF of each composition, the standard deviation (STD) of the SPF measurements, the percent increase or decrease (%) of each composition as compared to the control composition (Example 112) having no SPF booster, and the critical wavelength (CWL), in nm, of each composition.

Table 19

| EX# | SPF Booster | A | SPF | STD | % | CWL |
|---|---|---|---|---|---|---|
| 112 | N/A | 0 | 17.9 | 5.8 | N/A | 371.0 |
| 113 | Carnauba T3 | 5 | 30.3 | 8.6 | 69 | 369.8 |
| 114 | Carnauba T3 | 3 | 16.7 | 5.4 | -7 | 371.3 |
| 115 | Carnauba T3 | 1 | 12.8 | 2.4 | -28 | 372.0 |
| 116 | Synthetic | 5 | 25.6 | 10.0 | 43 | 370.1 |
| 117 | Synthetic | 3 | 20.3 | 4.2 | 13 | 371.7 |
| 118 | Synthetic | 1 | 14.0 | 3.3 | -21 | 372.0 |
| 119 | Rice Bran A | 5 | 27.2 | 8.1 | 52 | 370.5 |
| 120 | Rice Bran A | 3 | 13.9 | 1.1 | -22 | 372.0 |
| 121 | Rice Bran A | 1 | 15.9 | 3.2 | -11 | 372.0 |
| 122 | Rice Bran B | 5 | 24.8 | 5.3 | 38 | 370.4 |
| 123 | Rice Bran B | 3 | 17.9 | 3.3 | 0 | 372.0 |
| 124 | Rice Bran B | 1 | 14.7 | 2.5 | -18 | 372.0 |

Examples 125 through 138 were prepared with the ingredients and amounts listed in Table 20, below.

Table 20

| Ingredient INCI Name | Wt. % | Function |
|---|---|---|
| SPF Booster | 5.0 | SPF Booster |
| Deionized Water | QS To 100.0 | Diluent |
| Neopentyl Glycol Diethylhexanoate (Schercemol™ NGDO) | 5.0 | Emollient |
| Cetyl Octanoate (Schercemol™ CO Ester) | 15.0 | Emollient/Sensory Modifier |
| Isopropyl Isostearate (Schercemol™ 318 Ester) | 10.0 | Emollient/Sensory Modifier |
| Polyhydroxystearic Acid and Neopentyl Glycol Diethylhexanoate (Matrifuse™ S-1 Dispersant) | 1.0 | Dispersant/Stabilizer |
| Zinc Oxide (and) Triethoxycaprylylsilane | 10.0 | UV Filter |
| Titanium Dioxide (and) Aluminum Hydroxide (and) Stearic Acid | 5.0 | UV Filter |
| Polyurethane-62 (and) Trideceth-6 (Avalure™ Flex-6 Polymer) | 0.5 | Polymeric Emulsifier/ Film Former |

(continued)

| Ingredient INCI Name | Wt. % | Function |
|---|---|---|
| Sodium Chloride | 0.7 | Stabilizer |
| Phenoxyethanol (and) Ethylhexylglycerin | 0.5 | Preservative |

The compositions described in Table 20 were prepared according to the procedure described above with regard to the compositions described in Table 16.

All of Examples 125 through 138 were prepared at a processing temperature of 50 °C. The SPF booster used in each example, as well as the amount ("A", in wt.%) of SPF booster in each example, is listed in Table 21. Table 21 also lists the phase (P) of the composition to which the SPF booster was added (O=oil phase; W=water phase; PA = "post add"), the Sun Protection Factor (SPF), the post-photostability SPF (P-SPF), the post-water resistance SPF (W-SPF), and the standard deviation (STD) of each of the SPF measurements. "SA" refers to styrene/acrylate copolymer.

Table 21

| EX# | SPF Booster | A | P | SPF | STD | P-SPF | STD | W-SPF | STD |
|---|---|---|---|---|---|---|---|---|---|
| 125 | N/A | 0 | N/A | 17.9 | 5.8 | 20.7 | 5.0 | 10.9 | 1.3 |
| 126 | Carnauba T3 | 5 | O | 29.7 | 8.2 | 27.9 | 7.0 | 30.9 | 8.0 |
| 127 | Carnauba T3 | 3 | PA | 16.7 | 5.4 | 16.7 | 5.5 | 16.0 | 4.8 |
| 128 | Carnauba T3 | 1 | PA | 12.8 | 2.4 | 12.7 | 2.2 | 10.5 | 1.9 |
| 129 | Synthetic | 5 | O | 36.5 | 8.9 | 26.3 | 6.8 | 19.1 | 4.3 |
| 130 | Synthetic | 3 | PA | 20.3 | 4.2 | 20.2 | 4.2 | 12.1 | 2.1 |
| 131 | Synthetic | 1 | PA | 14.0 | 3.3 | 13.6 | 3.3 | 7.4 | 1.2 |
| 132 | Rice Bran A | 5 | O | 22.7 | 9.3 | 26.5 | 10.7 | 23.5 | 8.4 |
| 133 | Rice Bran A | 3 | PA | 13.9 | 1.1 | 13.9 | 1.0 | 12.1 | 1.0 |
| 134 | Rice Bran A | 1 | PA | 15.9 | 3.2 | 14.9 | 3.0 | 8.4 | 1.1 |
| 135 | Rice Bran B | 5 | O | 32.5 | 9.1 | 34.1 | 6.1 | 28.3 | 4.8 |
| 136 | Rice Bran B | 3 | PA | 17.9 | 3.3 | 18.0 | 3.4 | 15.3 | 2.6 |
| 137 | Rice Bran B | 1 | PA | 14.7 | 2.5 | 14.6 | 2.7 | 8.4 | 1.0 |
| 138 | SA | 5 | W | 38.5 | 15.0 | 29.8 | 6.1 | 14.0 | 2.4 |

Examples 139 through 148 were prepared with the ingredients and amounts listed in Table 22, below.

Table 22

| Ingredient INCI Name | Wt. % | Function |
|---|---|---|
| SPF Booster | 5.0 | SPF Booster |
| Deionized Water | QS To 100.0 | Diluent |
| Neopentyl Glycol Diethylhexanoate (Schercemol™ NGDO) | 5.0 | Emollient |
| Cetyl Octanoate (Schercemol™ CO Ester) | 15.0 | Emollient/Sensory Modifier |
| Isopropyl Isostearate (Schercemol™ 318 Ester) | 10.0 | Emollient/Sensory Modifier |
| Polyhydroxystearic Acid and Neopentyl Glycol Diethylhexanoate (Matrifuse™ S-1 Dispersant) | 1.0 | Dispersant/Stabilizer |
| Zinc Oxide (and) Triethoxycaprylylsilane | 10.0 | UV Filter |
| Titanium Dioxide (and) Aluminum Hydroxide (and) Stearic Acid | 5.0 | UV Filter |
| Polyurethane-62 (and) Trideceth-6 (Avalure™ Flex-6 Polymer) | 0.5 | Polymeric Emulsifier/ Film Former |

(continued)

| Ingredient INCI Name | Wt. % | Function |
|---|---|---|
| Sodium Chloride | 0.7 | Stabilizer |
| Phenoxyethanol (and) Ethylhexylglycerin | 0.5 | Preservative |

The compositions described in Table 22 were prepared according to the procedure described above with regard to the compositions described in Table 16.

All of Examples 139 through 148 were prepared at a processing temperature of 50 °C. The SPF boosters ("Booster 1" and "Booster 2") used in each example is listed in Table 23. All of the waxes of Examples 139 through 148 were "post added" to the compositions. Table 23 also lists the Sun Protection Factor (SPF), the post-photostability SPF (P-SPF), the post-water resistance SPF (W-SPF), and the standard deviation (STD) of each of the SPF measurements.

Table 23

| EX# | Booster 1 | Booster 2 | SPF | STD | P-SPF | STD | W-SPF | STD |
|---|---|---|---|---|---|---|---|---|
| 139 | N/A | N/A | 17.9 | 5.8 | 20.7 | 5.0 | 10.9 | 1.3 |
| 140 | Carnauba T3 | Rice Bran A | 29.4 | 4.1 | 29.0 | 4.4 | 35.3 | 5.7 |
| 141 | Carnauba T3 | Rice Bran B | 32.2 | 5.9 | 31.0 | 5.9 | 37.7 | 7.9 |
| 142 | Carnauba T3 | Castor | 25.5 | 3.1 | 25.0 | 3.1 | 30.1 | 7.7 |
| 143 | Rice Bran A | Rice Bran B | 28.7 | 4.1 | 30.0 | 5.4 | 28.9 | 4.8 |
| 144 | Rice Bran A | Castor | 26.3 | 3.5 | 26.9 | 3.7 | 27.4 | 4.3 |
| 145 | Rice Bran B | Castor | 27.8 | 3.3 | 30.8 | 3.5 | 28.7 | 3.8 |
| 146 | Carnauba T1 | Rice Bran A | 32.4 | 1.6 | 32.7 | 1.6 | 38.3 | 2.1 |
| 147 | Carnauba T1 | Rice Bran B | 35.5 | 2.99 | 34.8 | 3.1 | 44.9 | 3.3 |
| 148 | Carnauba T1 | Castor | 28.1 | 1.3 | 27.4 | 1.2 | 38.3 | 1.7 |

Examples 149 through 155 were prepared with the ingredients and amounts listed in Table 24, below.

Table 24

| Ingredient INCI Name | Wt. % | Function |
|---|---|---|
| SPF Booster | 5.0 | SPF Booster |
| Deionized Water | QS To 100.0 | Diluent |
| Neopentyl Glycol Diethylhexanoate (Schercemol™ NGDO) | 5.0 | Emollient |
| Cetyl Octanoate (Schercemol™ CO Ester) | 15.0 | Emollient/Sensory Modifier |
| Isopropyl Isostearate (Schercemol™ 318 Ester) | 10.0 | Emollient/Sensory Modifier |
| Polyhydroxystearic Acid and Neopentyl Glycol Diethylhexanoate (Matrifuse™ S-1 Dispersant) | 1.0 | Dispersant/Stabilizer |
| Zinc Oxide (and) Triethoxycaprylylsilane | 10.0 | UV Filter |
| Titanium Dioxide (and) Aluminum Hydroxide (and) Stearic Acid | 5.0 | UV Filter |
| Polyurethane-62 (and) Trideceth-6 (Avalure™ Flex-6 Polymer) | 0.5 | Polymeric Emulsifier/ Film Former |
| Sodium Chloride | 0.7 | Stabilizer |
| Phenoxyethanol (and) Ethylhexylglycerin | 0.5 | Preservative |

The compositions described in Table 24 were prepared according to the procedure described above with regard to the compositions described in Table 16.

All of Examples 149 through 155 were prepared at a processing temperature of 50 °C, and included carnauba wax as the SPF booster. All of the waxes of Examples 150 through 155 were "post added" to the compositions, except for Example 150, which was added to the oil phase. Table 25 lists the amount ("A", in wt.%) and grade ("G") of carnauba wax used, the Sun Protection Factor (SPF), the post-photostability SPF (P-SPF), the post-water resistance SPF (W-SPF), and the standard deviation (STD) of each of the SPF measurements.

Table 25

| EX# | A | G | SPF | STD | P-SPF | STD | W-SPF | STD |
|---|---|---|---|---|---|---|---|---|
| 149 | 0 | N/A | 17.9 | 5.8 | 20.7 | 5.0 | 10.9 | 1.3 |
| 150 | 5 | T3 | 29.7 | 8.2 | 27.9 | 7.0 | 30.9 | 8.0 |
| 151 | 3 | T3 | 16.7 | 5.4 | 16.7 | 5.5 | 16.0 | 4.8 |
| 152 | 1 | T3 | 12.8 | 2.4 | 12.7 | 2.2 | 10.5 | 1.9 |
| 153 | 5 | T1 | 37.7 | 2.6 | 37.1 | 2.1 | 41.5 | 3.4 |
| 154 | 3 | T1 | 40.5 | 2.1 | 42.2 | 3.3 | 34.2 | 2.1 |
| 155 | 1 | T1 | 37.6 | 3.6 | 47.7 | 3.1 | 29.3 | 2.5 |

It is noted that T1 grade carnauba wax seems to be a more efficient SPF booster in compositions including inorganic UV filters, while T3 grade carnauba wax seems to be more efficient in compositions including organic UV filters. Without wishing to be limited by theory, it is believed that, since inorganic UV filters block UV rays mostly through scattering and reflect, a lighter color SPF booster may work better. On the other hand, organic UV filters, which block UV rays mostly by absorption, a darker color SPF booster may work better.

Examples 156 and 157 were prepared according to the following descriptions, in order to explore the difference between preparation methods. Example 156 included the ingredients shown in Table 26, with the percentages reflecting percent by weight based on the total weight of the overall composition:

Table 26

| Phase | Ingredient | % |
|---|---|---|
| A | Water | 49.15 |
| A | Disodium EDTA | 0.1 |
| A | Propanediol (Zemea® from DuPont Tate & Lyle BioProducts) | 10 |
| A | Acrylates/$C_{10-30}$ Alkyl Acrylate Crosspolymer (Pemulen™ EZ-4U Polymeric Emulsifier) | 0.25 |
| B | Octocrylene | 10 |
| B | Homosalate | 10 |
| B | Ethylhexyl Salicylate | 5 |
| B | Butyl Methoxydibenzoyl Methane | 3 |
| B | Diisopropyl Sebacate (Schercemol™ DIS ester) | 6 |
| B | Polyglyceryl-3 Laurate (Hydramol™ TGL ester) | 0.5 |
| B | SPF Booster | 5 |
| C | Sodium Hydroxide 20% P/P | 0.5 |
| D | Preservative (Euxyl® PE 9010 from Schülke Inc.) | 0.5 |

In a clean, crystal beaker, the ingredients of Phase A (other than the Acrylates/$C_{10-30}$ Alkyl Acrylate Crosspolymer) were mixed under stirring, followed by addition of the Acrylates/$C_{10-30}$ Alkyl Acrylate Crosspolymer under strong stirring, followed by heating to 90 °C. In a separate clean, crystal beaker, the ingredients of Phase B were mixed under stirring while heating to 90 °C. These two mixtures were then combined under strong stirring, then homogenized for 1 minute at 10,000 rpm. The combined mixture was cooled to 30 °C, followed by neutralization with Phase C, and then addition of Phase D.

Example 157 included the same ingredients as Example 156, with the only difference being that the SPF Booster

was not included in Phase B. Rather, the SPF Booster was held out until the end of the mixing process of Example 156, at which time the SPF Booster was added under high shear (using an ULTRA-TURRAX® T 25 disperser from IKA at 10,000 RPM) for 2 minutes. Because the SPF Booster was held out until the end, it was not heated to 90 °C as in Example 156, which means that the SPF Booster of Example 157 was non-solubilized. The SPF Booster in Examples 156 and 157 was a mixture of carnauba wax and rice bran wax.

The in vitro SPF of Examples 156 and 157 were measured, with Example 156 having an SPF of about 23, while Example 157 had an SPF of about 35. This shows that the non-solubilized SPF Booster of Example 157 provides a distinct SPF increase over the solubilized SPF Booster of Example 156.

Particle sizes for each of the mixtures of Examples 156 and 157 were also measured, with the results shown in Table 27:

Table 27

| | D10 | D50 | D90 | D4,3 | D3,2 | D98 |
|---|---|---|---|---|---|---|
| Example 156 | 19.1 | 32.3 | 52.0 | 34.1 | 29.7 | 63.8 |
| Example 157 | 18.2 | 27.5 | 40.8 | 28.6 | 26.1 | 48.5 |

It is difficult to determine with certainty what impact the SPF Booster being solubilized versus non-solubilized had on the particle size, as there are many ingredients in each mixture which contribute to the particle size measurements. But nonetheless it is clear that there is a difference in particle size between these Examples 156 and 157. Without wishing to be limited by theory, it is believed that the difference in particle size may be largely attributed to the SPF Booster, since the ingredients and preparation methods between the two examples were otherwise the same, and that the difference in particle size at least partially contributed to the difference in SPF measurements between the two examples.

Each of the documents referred to above is incorporated herein by reference, including any prior applications, whether or not specifically listed above, from which priority is claimed. The mention of any document is not an admission that such document qualifies as prior art or constitutes the general knowledge of the skilled person in any jurisdiction. Except in the Examples, or where otherwise explicitly indicated or required by context, all numerical quantities in this description specifying amounts of materials, reaction conditions, molecular weights, number of carbon atoms, and the like, are to be understood as modified by the word "about". It is to be understood that the upper and lower amount, range, and ratio limits set forth herein may be independently combined, and that any amount within a disclosed range is contemplated to provide a minimum or maximum of a narrower range in alternative embodiments (with the proviso, of course, that the minimum amount of a range must be lower than the maximum amount of the same range). Similarly, the ranges and amounts for each element of the subject matter disclosed herein may be used together with ranges or amounts for any of the other elements.

While certain representative embodiments and details have been shown for the purpose of illustrating the subject matter disclosed herein, it will be apparent to those skilled in this art that various changes and modifications may be made therein without departing from the scope of the subject matter. In this regard, the scope of the invention is to be limited only by the following claims.

## Claims

**1.** A water-resistant and/or photoprotective composition comprising:

a. an aqueous phase comprising from 0% to 99.9% by weight of the composition, based on the total weight of the composition;
b. an oil phase comprising from 0% to 99.9% by weight of the composition, based on the total weight of the composition;
c. an active sun block agent; and
d. a micronized, non-solubilized wax comprising 0.1% to 10% by weight of the composition, based on the total weight of the composition;
wherein the wax has a D50 particle size of 1 to 100 μm, and a melting point of at least 70 °C; and
wherein the composition comprises at least 50% by weight of at least one of the aqueous phase or the oil phase.

**2.** The composition of claim 1, wherein the wax has a D50 particle size of 1 to 50 μm.

**3.** The composition of either claim 1 or claim 2, wherein the wax has a melting point of 70 to 100 °C.

**4.** The composition of any one of claims 1 to 3, wherein the wax comprises 1% to 5% of the composition, based on the total weight of the composition.

**5.** The composition of any one of claims 1 to 4, wherein the composition further comprises a stabilizing and/or emulsifying component.

**6.** The composition of claim 5, wherein the stabilizing and/or emulsifying component is present in the composition in an amount of 0.1% to 10% by weight, based on the total weight of the composition.

**7.** The composition of any one of claims 1 to 6, wherein the composition further comprises an artificial tanning agent.

**8.** The composition of any one of claims 1 to 7, formulated as a cream, dispersion, emulsion, gel, ointment, lotion, foam, spray, or tonic.

**9.** A topically-applicable cosmetic/dermatologic sunscreen oil-in-water emulsion composition for ultraviolet-photoprotection of skin, scalp, lips, mucous membranes, and/or hair against the damaging effects of ultraviolet irradiation comprising (a) an active sun block agent and (b) a micronized, non-solubilized natural wax, formulated into (c) a topically-applicable, cosmetically/dermatologically-acceptable vehicle, diluent or carrier.

**10.** A topically-applicable cosmetic/dermatologic formulation composition for ultraviolet-photoprotection of skin, scalp, lips, mucous membranes, and/or hair against the damaging effects of ultraviolet irradiation comprising (a) an active sun block agent and (b) a micronized, non-solubilized natural wax, formulated into (c) a topically-applicable, cosmetically/dermatologically-acceptable vehicle, diluent or carrier.

**11.** A water-resistant and/or photoprotective composition comprising:

a. an aqueous phase comprising from 20% to 80% by weight of the composition, based on the total weight of the composition;
b. an oil phase comprising from 5% to 50% by weight of the composition, based on the total weight of the composition, wherein the oil phase comprises an organic active sun block agent; and
c. a micronized, non-solubilized, natural wax comprising 1% to 5% by weight of the composition, based on the total weight of the composition; and
d. a stabilizing and/or emulsifying component comprising 1% to 10% by weight of the composition, based on the total weight of the composition;

wherein the wax has a D50 particle size of 2 to 50 $\mu$m, and a melting point of 70 to 100 °C.

**12.** A method of mitigating the effects of ultraviolet irradiation on human skin, scalp, lips, mucous membranes and/or hair by applying the composition of any one of claims 1 to 11 thereto, in an amount effective to at least partially mitigate the effects of ultraviolet irradiation.

**13.** Use of the composition of any one of claims 1 to 11 for ultraviolet-photoprotection of human skin, scalp, lips, mucous membranes and/or hair against the damaging effects of ultraviolet irradiation.

**14.** Use of a micronized, non-solubilized wax in a sunscreen composition for improving the aesthetics and/or sun protection factor of the sunscreen composition.

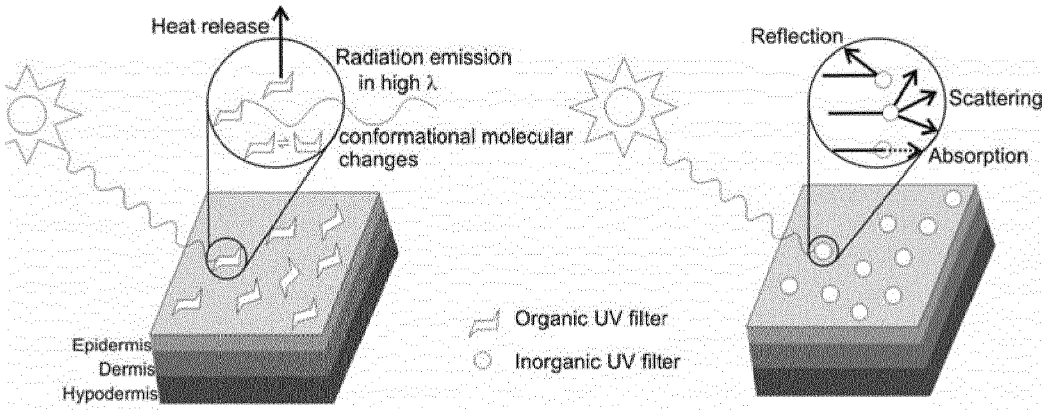


FIG. 1

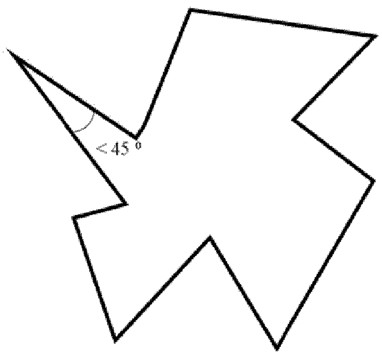


FIG. 2

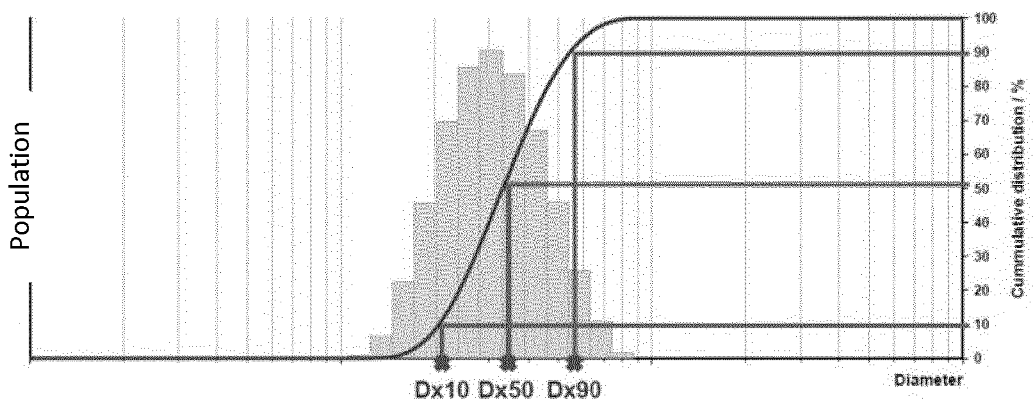


FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4585597 A
- US 20060292095 A1